# EUROPEAN PATENT SPECIFICATION

(11) **EP 1 412 289 B1**
(45) Date of publication and mention of the grant of the patent: **31.10.2007**
(21) Application number: 02763225.6
(22) Date of filing: 03.07.2002
(51) Int. Cl.: C01B 21/20, C10G 11/05, C01B 39/48, B01J 29/035, B01J 29/70, B01J 29/72, B01J 29/74, B01J 29/76, B01J 29/86, C01B 39/12, C10G 29/20, C10G 45/64, C10G 47/16

(54) **ZEOLITE SSZ-58**
ZEOLITH SSZ-58
ZEOLITHE SSZ-58

(30) Priority: 13.07.2001 US 905472; 13.07.2001 US 905352; 13.07.2001 US 905350
(43) Date of publication of application: 28.04.2004
(73) Proprietor: Chevron U.S.A. Inc., San Ramon, CA 94583 (US)
(72) Inventor: ELOMARI, Saleh, Richmond, CA 94804 (US)
(74) Representative: Nash, David Allan
(86) International application number: PCT/US2002/021172
(87) International publication number: WO 2003/006370

(56) References cited:
- WO-A-01/09037
- WO-A-01/44109
- WO-A-03/006365
- US-A- 4 941 963
- US-A- 6 049 018

## Description

### BACKGROUND OF THE INVENTION

### Field of the Invention

The present invention relates to new crystalline zeolite SSZ-58 and a method for preparing SSZ-58 using a N-butyl-N-cyclooctylpyrrolidinium cation or N-propylcyclooctylpyrrolidinium cation templating agent.

### State of the Art

Because of their unique sieving characteristics, as well as their catalytic properties, crystalline molecular sieves and zeolites are especially useful in applications such as hydrocarbon conversion, gas drying and separation. Although many different crystalline molecular sieves have been disclosed, there is a continuing need for new zeolites with desirable properties for gas separation and drying, hydrocarbon and chemical conversions, and other applications. New zeolites may contain novel internal pore architectures, providing enhanced selectivities in these processes.

Crystalline aluminosilicates are usually prepared from aqueous reaction mixtures containing alkali or alkaline earth metal oxides, silica, and alumina. Crystalline borosilicates are usually prepared under similar reaction conditions except that boron is used in place of aluminum. By varying the synthesis conditions and the composition of the reaction mixture, different zeolites can often be formed.

WO0109037 relates to crystalline zeolite SSZ-52 prepared using a quaternary ammonium cation templating agent comprising an anion which is not detrimental to the formation of the SSZ-52. SSZ-52 is useful in catalysts for hydrocarbon conversion reactions.

WO0144109 relates to crystalline zeolite SSZ-50 prepared using a quaternary ammonium cation templating agent comprising an anion which is not detrimental to the formation of the SSZ-50. SSZ-50 is useful in catalysts for hydrocarbon conversion reactions.

US6049018 relates to a synthetic porous crystalline material, designated MCM-68, a method and novel polycyclic organic cation for its preparation and its use in catalytic conversion of organic compounds. The crystalline material exhibits a distinctive X-ray diffraction pattern and has a unique crystal structure which contains at least one channel system, in which each channel is defined by a 12-membered ring of tetrahedrally coordinated atoms, and at least two further, independent channel systems, in each of which each channel is defined by a 10-membered ring of tetrahedrally coordinated atoms, wherein the number of unique 10-membered ring channels is twice the number of 12-membered ring channels.

### SUMMARY OF INVENTION

The present invention is directed to a family of crystalline molecular sieves with unique properties, referred to herein as "zeolite SSZ-58" or simply "SSZ-58". Preferably, SSZ-58 is obtained in its silicate, aluminosilicate, titanosilicate, vanadosilicate or borosilicate form. The term "silicate" refers to a zeolite having a high mole ratio of silicon oxide relative to aluminum oxide, preferably a mole ratio greater than 100, including zeolites comprised entirely of silicon oxide. As used herein, the term "aluminosilicate" refers to a zeolite containing both alumina and silica and the term "borosilicate" refers to a zeolite containing oxides of both boron and silicon.

In accordance with the broadest aspect of this invention, there is provided a zeolite having a mole ratio greater than about 20 of an oxide of a first tetravalent element to an oxide of a second tetravalent element different from said first tetravalent element, trivalent element, pentavalent element or mixture thereof and having, after calcination, the X-ray diffraction lines of Table II.

Further, in accordance with the broadest aspect of the invention, there is provided a zeolite having a mole ratio greater than about 20 of an oxide selected from silicon oxide, germanium oxide and mixtures thereof to an oxide selected from aluminum oxide, gallium oxide, iron oxide, boron oxide, titanium oxide, indium oxide, vanadium oxide and mixtures thereof.

In an embodiment of the present invention there is further provided a zeolite having a composition, as synthesized and in the anhydrous state, in terms of mole ratios as follows:

YO₂/W_{c}O_{d} 20-∞

M_{2/n}/YO₂ 0.01 - 0.03

Q/YO₂ 0.02 - 0.05

wherein Y is silicon, germanium or a mixture thereof; W is aluminum, gallium, iron, boron, titanium, indium, vanadium or mixtures thereof; c is 1 or 2; d is 2 when c is 1 (i.e., W is tetravalent) or d is 3 or 5 when c is 2 (i.e., d is 3 when W is trivalent or 5 when W is pentavalent); M is an alkali metal cation, alkaline earth metal cation or mixtures thereof; n is the valence of M (i.e., 1 or 2); and Q is a N-butyl-N-cyclooctylpyrrolidinium cation or N-propyl-cyclooctylpyrrolidinium cation.

The zeolite of the present invention may be prepared by thermally treating a zeolite having a mole ratio of an oxide selected from silicon oxide, germanium oxide and mixtures thereof to an oxide selected from aluminum oxide, gallium oxide, iron oxide, boron oxide, titanium oxide, indium oxide, vanadium oxide and mixtures thereof greater than about 20 at a temperature of from about 200°C to about 800°C. The present invention also includes this thus-prepared zeolite which is predominantly in the hydrogen form, which hydrogen form is prepared by ion exchanging with an acid or with a solution of an ammonium salt followed by a second calcination.

Also provided in accordance with the present invention is a method of preparing the the zeolite described above, said method comprising contacting under crystallization conditions sources of said oxides and a templating agent comprising a N-butyl-N-cyclooctylpyrrolidinium cation or N-propyl-cyclooctylpyrrolidinium cation.

The present invention provides a process for converting hydrocarbons comprising contacting a hydrocarbonaceous feed at hydrocarbon converting conditions with a catalyst comprising the zeolite of this invention. The zeolite may be predominantly in the hydrogen form. It may also be substantially free of acidity.

Further provided by the present invention is a hydrocracking process comprising contacting a hydrocarbon feedstock under hydrocracking conditions with a catalyst comprising the zeolite of this invention, preferably predominantly in the hydrogen form.

This invention also includes a dewaxing process comprising contacting a hydrocarbon feedstock under dewaxing conditions with a catalyst comprising the zeolite of this invention, preferably predominantly in the hydrogen form.

The present invention also includes a process for improving the viscosity index of a dewaxed product of waxy hydrocarbon feeds comprising contacting the waxy hydrocarbon feed under, isomerization dewaxing conditions with a catalyst comprising the zeolite of this invention, preferably predominantly in the hydrogen form.

The present invention further includes a process for producing a C₂₀₊ lube oil from a C₂₀₊ olefin feed comprising isomerizing said olefin feed under isomerization conditions over a catalyst comprising at least one Group VIII metal and the zeolite of this invention. The zeolite may be predominantly in the hydrogen form.

In accordance with this invention, there is also provided a process for catalytically dewaxing a hydrocarbon oil feedstock boiling above about 177°C (350°F) and containing straight chain and slightly branched chain hydrocarbons comprising contacting said hydrocarbon oil feedstock in the presence of added hydrogen gas at a hydrogen pressure of about 0.10-20.68 MPa (15-3000 psi) with a catalyst comprising at least one Group VIII metal and the zeolite of this invention, preferably predominantly in the hydrogen form. The catalyst may be a layered catalyst comprising a first layer comprising at least one Group VIII metal and the zeolite of this invention, and a second layer comprising an aluminosilicate zeolite which is more shape selective than the zeolite of said first layer.

Also included in the present invention is a process for preparing a lubricating oil which comprises hydrocracking in a hydrocracking zone a hydrocarbonaceous feedstock to obtain an effluent comprising a hydrocracked oil, and catalytically dewaxing said effluent comprising hydrocracked oil at a temperature of at least about 204°C (400°F) and at a pressure of from about 0.103 MPa gauge (15 psig) to about 20.7 MPa gauge (3000 psig) in the presence of added hydrogen gas with a catalyst comprising at least one Group VIII metal and the zeolite of this invention. The zeolite may be predominantly in the hydrogen form.

Further included in this invention is a process for isomerization dewaxing a raffinate comprising contacting said raffinate in the presence of added hydrogen with a catalyst comprising at least one Group VIII metal and the zeolite of this invention. The raffinate may be bright stock, and the zeolite may be predominantly in the hydrogen form.

Also included in this invention is a process for increasing the octane of a hydrocarbon feedstock to produce a product having an increased aromatics content comprising contacting a hydrocarbonaceous feedstock which comprises normal and slightly branched hydrocarbons having a boiling range above about 40°C and less than about 200°C, under aromatic conversion conditions with a catalyst comprising the zeolite of this invention made substantially free of acidity by neutralizing said zeolite with a basic metal. Also provided in this invention is such a process wherein the zeolite contains a Group VIII metal component.

Also provided by the present invention is a catalytic cracking process comprising contacting a hydrocarbon feedstock in a reaction zone under catalytic cracking conditions in the absence of added hydrogen with a catalyst comprising the zeolite of this invention, preferably predominantly in the hydrogen form. Also included in this invention is such a catalytic cracking process wherein the catalyst additionally comprises a large pore crystalline cracking component.

This invention further provides an isomerization process for isomerizing C₄ to C₇ hydrocarbons, comprising contacting a feed having normal and slightly branched C₄ to C₇ hydrocarbons under isomerizing conditions with a catalyst comprising the zeolite of this invention, preferably predominantly in the hydrogen form. The zeolite may be impregnated with at least one Group VIII metal, preferably platinum. The catalyst may be calcined in a steam/air mixture at an elevated temperature after impregnation of the Group VIII metal.

Also provided by the present invention is a process for alkylating an aromatic hydrocarbon which comprises contacting under alkylation conditions at least a molar excess of an aromatic hydrocarbon with a C₂ to C₂₀ olefin under at least partial liquid phase conditions and in the presence of a catalyst comprising the zeolite of this invention, preferably predominantly in the hydrogen form. The olefin may be a C₂ to C₄ olefin, and the aromatic hydrocarbon and olefin may be present in a molar ratio of about 4:1 to about 20:1, respectively. The aromatic hydrocarbon may be selected from the group consisting of benzene, toluene, ethylbenzene, xylene, naphthalene, naphthalene derivatives, such as dimethylnaphthalene, or mixtures thereof.

Further provided in accordance with this invention is a process for transalkylating an aromatic hydrocarbon which comprises contacting under transalkylating conditions an aromatic hydrocarbon with a polyalkyl aromatic hydrocarbon under at least partial liquid phase conditions and in the presence of a catalyst comprising the zeolite of this invention, preferably predominantly in the hydrogen form. The aromatic hydrocarbon and the polyalkyl aromatic hydrocarbon may be present in a molar ratio of from about 1:1 to about 25:1, respectively.

The aromatic hydrocarbon may be selected from the group consisting of benzene, toluene, ethylbenzene, xylene, or mixtures thereof, and the polyalkyl aromatic hydrocarbon may be a dialkylbenzene.

Further provided by this invention is a process to convert paraffins to aromatics which comprises contacting paraffins under conditions which cause paraffins to convert to aromatics with a catalyst comprising the zeolite of this invention, said catalyst comprising gallium, zinc, or a compound of gallium or zinc.

In accordance with this invention there is also provided a process for isomerizing olefins comprising contacting said olefin under conditions which cause isomerization of the olefin with a catalyst comprising the zeolite of this invention.

Further provided in accordance with this invention is a process for isomerizing an isomerization feed comprising an aromatic C₈ stream of xylene isomers or mixtures of xylene isomers and ethylbenzene, wherein a more nearly equilibrium ratio of ortho-, meta- and para-xylenes is obtained, said process comprising contacting said feed under isomerization conditions with a catalyst comprising the zeolite of this invention.

The present invention further provides a process for oligomerizing olefins comprising contacting an olefin feed under oligomerization conditions with a catalyst comprising the zeolite of this invention.

This invention also provides a process for converting lower alcohols and other oxygenated hydrocarbons comprising contacting said lower alcohol or other oxygenated hydrocarbon with a catalyst comprising the zeolite of this invention under conditions to produce liquid products.

Further provided in accordance with the present invention is a process for the production of higher molecular weight hydrocarbons from lower molecular weight hydrocarbons comprising the steps of:
(a) introducing into a reaction zone a lower molecular weight hydrocarbon-containing gas and contacting said gas in said zone under C₂₊ hydrocarbon synthesis conditions with the catalyst and a metal or metal compound capable of converting the lower molecular weight hydrocarbon to a higher molecular weight hydrocarbon; and
(b) withdrawing from said reaction zone a higher molecular weight hydrocarbon-containing stream.

In accordance with the present invention, there is provided a process for the reduction of oxides of nitrogen contained in a gas stream in the presence of oxygen wherein said process comprises contacting the gas stream with the zeolite of the present invention. The zeolite may contain a metal or metal ions (such as cobalt, copper or mixtures thereof) capable of catalyzing the reduction of the oxides of nitrogen, and may be conducted in the presence of a stoichiometric excess of oxygen. In a preferred embodiment, the gas stream is the exhaust stream of an internal combustion engine.

### DETAILED DESCRIPTION OF THE INVENTION

The present invention comprises a family of crystalline, zeolites designated herein "zeolite SSZ-58" or simply "SSZ-58". SSZ-58 is believed to be a large pore zeolite. As used herein, the term "large pore" means having an average pore size diameter greater than about 6.5 Angstroms (6.5 x 10⁻¹⁰ m), preferably from about 7 Angstroms (7 x 10⁻¹⁰ m) about 8 Angstroms (8 x 10⁻¹⁰ m).

In preparing SSZ-58 zeolites, a N-butyl-N-cyclooctylpyrrolidinium cation or N-propyl-cyclooctylpyrrolidinium cation is used as a crystallization template. In general, SSZ-58 is prepared by contacting an active source of one or more oxides selected from the group consisting of monovalent element oxides, divalent element oxides, trivalent element oxides, and tetravalent element oxides with the templating agent.

SSZ-58 is prepared from a reaction mixture having the composition shown in Table A below.

**TABLE A**

| | Reaction Mixture | |
|---|---|---|
| | Typical | Preferred |
| YO₂/WₐO_{b} | >20 | 35 - 65 |
| OH-/YO₂ | 0.10 - 0.50 | 0.15 - 0.25 |
| Q/YO₂ | 0.05 - 0.50 | 0.10 - 0.20 |
| M_{2/n}/YO₂ | 0.02 - 0.40 | 0.10 - 0.30 |
| H₂O/YO₂ | 25 - 100 | 30 - 50 |

where Y, W, Q, M and n are as defined above, and a is 1 or 2, and b is 2 when a is 1 (i.e., W is tetravalent) and b is 3 when a is 2 (i.e., W is trivalent).

In practice, SSZ-58 is prepared by a process comprising:
(a) preparing an aqueous solution containing sources of at least one oxide capable of forming a crystalline molecular sieve and a N-butyl-N-cyclooctylpyrrolidinium cation or N-propyl-cyclooctylpyrrolidinium cation having an anionic counterion which is not detrimental to the formation of SSZ-58;
(b) maintaining the aqueous solution under conditions sufficient to form crystals of SSZ-58; and
(c) recovering the crystals of SSZ-58.

Accordingly, SSZ-58 may comprise the crystalline material and the templating agent in combination with metallic and non-metallic oxides bonded in tetrahedral coordination through shared oxygen atoms to form a cross-linked three dimensional crystal structure. The metallic and non-metallic oxides comprise one or a combination of oxides of a first tetravalent element(s), and one or a combination of a second tetravalent element(s) different from the first tetravalent element(s), trivalent element(s), pentavalent element(s) or mixture thereof. The first tetravalent element(s) is preferably selected from the group consisting of silicon, germanium and combinations thereof. More preferably, the first tetravalent element is silicon. The second tetravalent element (which is different from the first tetravalent element), trivalent element and pentavalent element is preferably selected from the group consisting of aluminum, gallium, iron, boron, titanium, indium, vanadium and combinations thereof. More preferably, the second trivalent or tetravalent element is aluminum or boron.

Typical sources of aluminum oxide for the reaction mixture include aluminates, alumina, aluminum colloids, aluminum oxide coated on silica sol, hydrated alumina gels such as Al(OH)₃ and aluminum compounds such as AlCl₃ and Al₂(SO₄)₃. Typical sources of silicon oxide include silicates, silica hydrogel, silicic acid, fumed silica, colloidal silica, tetra-alkyl orthosilicates, and silica hydroxides. Boron, as well as gallium, germanium, titanium, indium, vanadium and iron, can be added in forms corresponding to their aluminum and silicon counterparts.

A source zeolite reagent may provide a source of aluminum or boron. In most cases, the source zeolite also provides a source of silica. The source zeolite in its dealuminated or deboronated form may also be used as a source of silica, with additional silicon added using, for example, the conventional sources listed above. Use of a source zeolite reagent as a source of alumina for the present process is more completely described in U.S. Patent No. 5,225,179, issued July 6, 1993 to Nakagawa entitled "Method of Making Molecular Sieves".

Typically, an alkali metal hydroxide and/or an alkaline earth metal hydroxide, such as the hydroxide of sodium, potassium, lithium, cesium, rubidium, calcium, and magnesium, is used in the reaction mixture; however, this component can be omitted so long as the equivalent basicity is maintained. The templating agent may be used to provide hydroxide ion. Thus, it may be beneficial to ion exchange, for example, the halide for hydroxide ion, thereby reducing or eliminating the alkali metal hydroxide quantity required. The alkali metal cation or alkaline earth cation may be part of the as-synthesized crystalline oxide material, in order to balance valence electron charges therein.

The reaction mixture is maintained at an elevated temperature until the crystals of the SSZ-58 zeolite are formed. The hydrothermal crystallization is usually conducted under autogenous pressure, at a temperature between 100°C and 200°C, preferably between 135°C and 160°C. The crystallization period is typically greater than 1 day and preferably from about 3 days to about 20 days.

Preferably, the zeolite is prepared using mild stirring or agitation.

During the hydrothermal crystallization step, the SSZ-58 crystals can be allowed to nucleate spontaneously from the reaction mixture. The use of SSZ-58 crystals as seed material can be advantageous in decreasing the time necessary for complete crystallization to occur. In addition, seeding can lead to an increased purity of the product obtained by promoting the nucleation and/or formation of SSZ-58 over any undesired phases. When used as seeds, SSZ-58 crystals are added in an amount between 0.1 and 10% of the weight of silica used in the reaction mixture.

Once the zeolite crystals have formed, the solid product is separated from the reaction mixture by standard mechanical separation techniques such as filtration. The crystals are water-washed and then dried, e.g., at 90°C to 150°C for from 8 to 24 hours, to obtain the as-synthesized SSZ-58 zeolite crystals. The drying step can be performed at atmospheric pressure or under vacuum.

SSZ-58 as prepared has a mole ratio of an oxide selected from silicon oxide, germanium oxide and mixtures thereof to an oxide selected from aluminum oxide, gallium oxide, iron oxide, boron oxide, titanium oxide, indium oxide, vanadium oxide and mixtures thereof greater than about 20; and has, after calcination, the X-ray diffraction lines of Table II below. SSZ-58 further has a composition, as synthesized (i.e., prior to removal of the templating agent from the zeolite) and in the anhydrous state, in terms of mole ratios, shown in Table B below.

**TABLE B**

| As-Synthesized SSZ-58 | |
|---|---|
| YO₂/W_{c}O_{d} | >20 |
| M_{2/n}/YO₂ | 0.01-0.03 |
| Q/YO₂ | 0.02 - 0.05 |

where Y, W, c, d, M, n and Q are as defined above.

SSZ-58 can be made essentially aluminum free, i.e., having a silica to alumina mole ratio of ∞. A method of increasing the mole ratio of silica to alumina is by using standard acid leaching or chelating treatments. However, essentially aluminum-free SSZ-58 can be synthesized directly using essentially aluminum-free silicon sources as the main tetrahedral metal oxide component, if boron is also present. SSZ-58 can also be prepared directly as either an aluminosilicate or a borosilicate.

Lower silica to alumina ratios may also be obtained by using methods which insert aluminum into the crystalline framework. For example, aluminum insertion may occur by thermal treatment of the zeolite in combination with an alumina binder or dissolved source of alumina. Such procedures are described in U.S. Patent No. 4,559,315, issued on December 17, 1985 to Chang et al.

It is believed that SSZ-58 is comprised of a new framework structure or topology which is characterized by its X-ray diffraction pattern. SSZ-58 zeolites, as-synthesized, have a crystalline structure whose X-ray powder diffraction pattern exhibit the characteristic lines shown in Table I and is thereby distinguished from other zeolites.

**TABLE I**

| As-Synthesized SSZ-58 | | |
|---|---|---|
| 2 Theta (deg.)^{(a)} | d (Å/x10⁻¹⁰m) | Relative Intensity ^{(b)} |
| 7.1 | 12.4 | S |
| 7.7 | 11.5 | M |
| 9.9 | 8.93 | M |
| 10.5 | 8.42 | W |
| 12.1 | 7.31 | M |
| 17.3 | 5.12 | W |
| 19.7 | 4.50 | M |
| 21.0 | 4.23 | S |
| 21.9 | 4.06 | M |
| 22.35 | 3.97 | VS |

| | | |
|---|---|---|
| ^{(a)} ± 0.15 ^{(b)} The X-ray patterns provided are based on a relative intensity scale in which the strongest line in the X-ray pattern is assigned a value of 100: W(weak) is less than 20; M(medium) is between 20 and 40; S(strong) is between 40 and 60; VS(very strong) is greater than 60. | | |

Table IA below shows the X-ray powder diffraction lines for as-synthesized SSZ-58 including actual relative intensities.

**TABLE IA**

| As-Synthesized SSZ-58 | | |
|---|---|---|
| 2 Theta (deg.)^{(a)} | d (Å/ x 10⁻¹⁰m) | I/I₀ x 100 |
| 6.90 | 12.80 (Sh) | 6 |
| 7.06 | 12.51 | 39 |
| 7.72 | 1.44 | 16 |
| 9.86 | 8.963 (Sh) | 10 |
| 9.96 | 8.874 | 13 |
| 10.46 | 8.450 | 10 |
| 12.10 | 7.309 | 18 |
| 14.06 | 6.294 | 9 |
| 14.21 | 6.228 (Sh) | 7 |
| 15.46 | 5.727 | 5. |
| 15.68 | 5.647 | 6 |
| 16.12 | 5.494 | 4 |
| 17.24 | 5.139 | 14 |
| 17.36 | 5.104 (Sh) | 7 |
| 18.76 | 4.726 | 15 |
| 18.92 | 4.687 | 16 |
| 19.72 | 4.498 | 30 |
| 20.22 | 4.388 | 14 |
| 20.70 | 4.288 | 16 |
| 21.00 | 4.227 | 63 |
| 21.16 | 4.195 | 14 |
| 21.26 | 4.17.6 (Sh) | 12 |
| 21.88 | 4.059 | 26 |
| 22.28 | 3.987 (Sh) | 61 |
| 22.24 | 3.962 | 100 |
| 22.66 | 3.921 | 26 |
| 23.02 | 3.860 | 9 |
| 23.28 | 3.818 | 5 |
| 23.50 | 3.783 | 17 |
| 23.68 | 3.754 | 13 |
| 24.34 | 3.654 | 5 |
| 25.12 | 3.542 | 11 |
| 25.54 | 3.485 | 7 |
| 25.72 | 3.461 (Sh) | 4 |
| 26.12 | 3.409 | 8 |
| 26.58 | 3.351 | 7 |
| 27.30 | 3.264 | 11 |
| 27.58 | 3.232 | 7 |
| 27.94 | 3.191 | 5 |
| 28.50 | 3.129 (Sh) | 8 |
| 28.62 | 3.117 | 11 |
| 29.18 | 3.058 | 2 |
| 29.86 | 2.990 | 5 |
| 30.08 | 2.968 | 5 |
| 30.88 | 2.894 | 3 |
| 31.46 | 2.842 | 2 |
| 31.74 | 2.817 | 4 |
| 32.48 | 2.755 | 1 |
| 32.59 | 2.746 | 2 |
| 32.76 | 2.732 | 3 |
| 33.14 | 2.701 | 4 |
| 33.56 | 2.668 | 3 |
| 33.80 | 2.650 | 2 |
| 34.82 | 2.574 | 2 |
| 35.12 | 2.553 | 1 |
| 35.38 | 2.535 | 3 |
| 35.82 | 2.505 | 6 |
| 36.50 | 2.460 | 6 |
| 37.74 | 2.382 | 4 |
| 37.94 | 2.370 (Sh) | 2 |
| 38.44 | 2.340 | 2 |
| 39.29 | 2.291 | 2 |
| 39.62 | 2.273 | 1 |
| 41.10 | 2.194 | 1 |
| 43.12 | 2.096 | 2 |
| 43.30 | 2.086 | 5 |
| 43.50 | 2.079 | 2 |

| | | |
|---|---|---|
| ^{(a)}± 0.15 | | |

After calcination, the SSZ-58 zeolites have a crystalline structure whose X-ray powder diffraction pattern include the characteristic lines shown in Table II:

**TABLE II**

| Calcined SSZ-58 | | |
|---|---|---|
| 2 Theta (deg.)^{(a)} | d(Å/x10⁻¹⁰m) | Relative Intensity |
| 7.1 | 12.4 | VS |
| 7.7 | 11.5 | M |
| 9.9 | 8.93 | M |
| 10.5 | 8.42 | M |
| 12.1 | 7.31 | W |
| 17.3 | 5.12 | W |
| 19.8 | 4.48 | M |
| 21.0 | 4.23 | S |
| 21.9 | 4.06 | M |
| 22.4 | 3.97 | S |

| | | |
|---|---|---|
| ^{(a)}± 0.15 | | |

Table IIA below shows the X-ray powder diffraction lines for calcined SSZ-58 including actual relative intensities.

**TABLE IIA**

| Calcined SSZ-58 | | |
|---|---|---|
| Two Theta (deg.)^{(a)} | d(Å/x10⁻¹⁰m) | I/Io x 100 |
| 6.88 | 12.84 (Sh) | 17 |
| 7.06 | 12.51 | 100 |
| 7.70 | 11.47 | 22 |
| 9.86 | 8.963 (Sh) | 20 |
| 9.98 | 8.856 | 35 |
| 10.48 | 8.435 | 15 |
| 12.12 | 7.297 | 9 |
| 14.20 | 6.232 | 11 |
| 15.48 | 5.720 | 6 |
| 15.70 | 5.640 | 10 |
| 15.84 | 5.590 | 7 |
| 16.14 | 5.487 | 6 |
| 17.24 | 5.139 | 11 |
| 17.37 | 5.101 | 4 |
| 18.78 | 4.721 | 7 |
| 18.96 | 4.677 | 14 |
| 19.76 | 4.489 | 23 |
| 20.26 | 4.380 | 8 |
| 20.70 | 4.287 | 13 |
| 21.02 | 4.223 | 40 |
| 21.22 | 4.184 (Sh) | 9 |
| 21.90 | 4.055 | 18 |
| 22.35 | 3.975 (Sh) | 39 |
| 22.46 | 3.955 | 64 |
| 22.70 | 3.914 | 18 |
| 23.04 | 3.857 | 3 |
| 23.28 | 3.818 | 3 |
| 23.54 | 3.776 | 13 |
| 23.74 | 3.745 | 8 |
| 24.38 | 3.648 | 3 |
| 25.16 | 3.537 | 8 |
| 25.60 | 3.477 | 5 |
| 25.78 | 3.453 (Sh) | 4 |
| 26.14 | 3.406 | 5 |
| 26.64 | 3.343 | 6 |
| 27.34 | 3.259 | 6 |
| 27.64 | 3.225 | 6 |
| 27.98 | 3.186 | 4 |
| 28.58 | 3.121 (Sh) | 7 |
| 28.68 | 3.110 | 8 |
| 29.20 | 3.056 | 1 |
| 29.88 | 2.988 | 4 |
| 30.19 | 2.958 | 3 |
| 30.92 | 2.890 | 2 |
| 31.48 | 2.840 | 2 |
| 31.74 | 2.817 | 3 |
| 32.54 | 2.750 | 1 |
| 32.76 | 2.731 | 1 |
| 33.18 | 2.698 | 2 |
| 33.62 | 2.664 | 2 |
| 33.86 | 2.645 | 2 |
| 34.88 | 2.570 | 1 |
| 35.20 | 2.548 | 1 |
| 35.42 | 2.532 | 2 |
| 35.90 | 2.499 | 5 |
| 36.54 | 2.457 | 4 |
| 37.80 | 2.378 | 3 |
| 38.00 | 2.366 (Sh) | 2 |
| 38.50 | 2.336 | 1 |
| 39.30 | 2.291 | 1 |
| 43.20 | 2.092 | 2 |
| 43.42 | 2.082 | 4 |
| 43.53 | 2.077 | 3 |

The X-ray powder diffraction patterns were determined by standard techniques. The radiation was the K-alpha/doublet of copper. The peak heights and the positions, as a function of 28 where θ is the Bragg angle, were read from the relative intensities of the peaks, and d, the interplanar spacing in Angstroms (x10⁻¹⁰m) corresponding to the recorded lines, can be calculated.

The variation in the scattering angle (two theta) measurements, due to instrument error and to differences between individual samples, is estimated at ± 0.20 degrees.

The X-ray diffraction pattern of Table I is representative of "as-synthesized" or "as-made" SSZ-58 zeolites. Minor variations in the diffraction pattern can result from variations in the silica-to-alumina or silica-to-boron mole ratio of the particular sample due to changes in lattice constants. In addition, sufficiently small crystals will affect the shape and intensity of peaks, leading to significant peak broadening.

Representative peaks from the X-ray diffraction pattern of calcined SSZ-58 are shown in Table II. Calcination can also result in changes in the intensities of the peaks as compared to patterns of the "as-made" material, as well as minor shifts in the diffraction pattern. The zeolite produced by exchanging the metal or other cations present in the zeolite with various other cations (such as H⁺ or NH₄⁺) yields essentially the same diffraction pattern, although again, there may be minor shifts in the interplanar spacing and variations in the relative intensities of the peaks. Notwithstanding these minor perturbations, the basic crystal lattice remains unchanged by these treatments.

Crystalline SSZ-58 can be used as-synthesized, but preferably will be thermally treated (calcined). Usually, it is desirable to remove the alkali metal cation by ion exchange and replace it with hydrogen, ammonium, or any desired metal ion. The zeolite can be leached with chelating agents, e.g., EDTA or dilute acid solutions, to increase the silica to alumina mole ratio. The zeolite can also be steamed; steaming helps stabilize the crystalline lattice to attack from acids.

The zeolite can be used in intimate combination with hydrogenating components, such as tungsten, vanadium molybdenum, rhenium, nickel cobalt, chromium, manganese, or a noble metal, such as palladium or platinum, for those applications in which a hydrogenation-dehydrogenation function is desired.

Metals may also be introduced into the zeolite by replacing some of the cations in the zeolite with metal cations via standard ion exchange techniques (see, for example, U.S. Patent Nos. 3,140,249 issued July 7, 1964 to Plank et al.; 3,140,251 issued July 7, 1964 to Plank et al.; and 3,140,253 issued July 7, 1964 to Plank et al.). Typical replacing cations can include metal cations, e.g., rare earth, Group IA, Group IIA and Group VIII metals, as well as their mixtures. Of the replacing metallic cations, cations of metals such as rare earth, Mn, Ca, Mg, Zn, Cd, Pt, Pd, Ni, Co, Ti, Al, Sn, and Fe are particularly preferred.

The hydrogen, ammonium, and metal components can be ion-exchanged into the SSZ-58. The zeolite can also be impregnated with the metals, or, the metals can be physically and intimately admixed with the zeolite using standard methods known to the art.

Typical ion-exchange techniques involve contacting the synthetic zeolite with a solution containing a salt of the desired replacing cation or cations. Although a wide variety of salts can be employed, chlorides and other halides, acetates, nitrates, and sulfates are particularly preferred. The zeolite is usually calcined prior to the ion-exchange procedure to remove the organic matter present in the channels and on the surface, since this results in a more effective ion exchange. Representative ion exchange techniques are disclosed in a wide variety of patents including U.S. Patent Nos. 3,140,249 issued on July 7, 1964 to Plank et al.; 3,140,251 issued on July 7, 1964 to Plank et al.; and 3,140,253 issued on July 7, 1964 to Plank et al.

Following contact with the salt solution of the desired replacing cation, the zeolite is typically washed with water and dried at temperatures ranging from 65°C to about 200°C. After washing, the zeolite can be calcined in air or inert gas at temperatures ranging from about 200°C to about 800°C for periods of time ranging from 1 to 48 hours, or more, to produce a catalytically active product especially useful in hydrocarbon conversion processes.

Regardless of the cations present in the synthesized form of SSZ-58, the spatial arrangement of the atoms which form the basic crystal lattice of the zeolite remains essentially unchanged.

SSZ-58 can be formed into a wide variety of physical shapes. Generally speaking, the zeolite can be in the form of a powder, a granule, or a molded product, such as extrudate having a particle size sufficient to pass through a 2-mesh (Tyler) screen and be retained on a 400-mesh (Tyler) screen. In cases where the catalyst is molded, such as by extrusion with an organic binder, the aluminosilicate can be extruded before drying, or, dried or partially dried and then extruded.

SSZ-58 can be composited with other materials resistant to the temperatures and other conditions employed in organic conversion processes. Such matrix materials include active and inactive materials and synthetic or naturally occurring zeolites as well as inorganic materials such as clays, silica and metal oxides. Examples of such materials and the manner in which they can be used are disclosed in U.S. Patent No. 4,910,006, issued May 20, 1990 to Zones et al., and U.S. Patent No. 5,316,753, issued May 31, 1994 to Nakagawa.

### Hydrocarbon Conversion Processes

SSZ-58 zeolites are useful in hydrocarbon conversion reactions. Hydrocarbon conversion reactions are chemical and catalytic processes in which carbon containing compounds are changed to different carbon containing compounds. Examples of hydrocarbon conversion reactions in which SSZ-58 are expected to be useful include hydrocracking, dewaxing, catalytic cracking and olefin and aromatics formation reactions. The catalysts are also expected to be useful in other petroleum refining and hydrocarbon conversion reactions such as isomerizing n-paraffins and naphthenes, polymerizing and oligomerizing olefinic or acetylenic compounds such as isobutylene and butene-1, reforming, isomerizing polyalkyl substituted aromatics (e.g., m-xylene), and disproportionating aromatics (e.g., toluene) to provide mixtures of benzene, xylenes and higher methylbenzenes and oxidation reactions. Also included are rearrangement reactions to make various naphthalene derivatives, and forming higher molecular weight hydrocarbons from lower molecular weight hydrocarbons (e.g., methane upgrading). The SSZ-58 catalysts may have high selectivity, and under hydrocarbon conversion conditions can provide a high percentage of desired products relative to total products.

SSZ-58 zeolites can be used in processing hydrocarbonaceous feedstocks. Hydrocarbonaceous feedstocks contain carbon compounds and can be from many different sources, such as virgin petroleum fractions, recycle petroleum fractions, shale oil, liquefied coal, tar sand oil, synthetic paraffins from NAO, recycled plastic feedstocks and, in general, can be any carbon containing feedstock susceptible to zeolitic catalytic reactions. Depending on the type of processing the hydrocarbonaceous feed is to undergo, the feed can contain metal or be free of metals, it can also have high or low nitrogen or sulfur impurities. It can be appreciated, however, that in general processing will be more efficient (and the catalyst more active) the lower the metal, nitrogen, and sulfur content of the feedstock.

The conversion of hydrocarbonaceous feeds can take place in any convenient mode, for example, in fluidized bed, moving bed, or fixed bed reactors depending on the types of process desired. The formulation of the catalyst particles will vary depending on the conversion process and method of operation.

Other reactions which can be performed using the catalyst of this invention containing a metal, e.g., a Group VIII metal such platinum, include hydrogenation-dehydrogenation reactions, denitrogenation and desulfurization reactions.

The following table indicates typical reaction conditions which may be employed when using catalysts comprising SSZ-58 in the hydrocarbon conversion reactions of this invention. Preferred conditions are indicated in parentheses.

| Process | Temp.,°C | Pressure | LHSV |
|---|---|---|---|
| Hydrocracking | 175-485 | 0.5-350 bar | 0.1-30 |
| Dewaxing | 200-475 | 15-3000 psig, 0.103-20.7 MPa gauge (200-3000, 1.38-20.7 MPa gauge) | 0.1-20 |
| | (250-450) | | (0.2-10) |
| Aromatics formation | 400-600 | atm.-10 bar | 0.1-15 |
| | (480-550) | | |
| Cat. Cracking | 127-885 | subatm.-¹ (atm.-5 atm.) | 0.5-50 |
| Oligomerization | 232-649² | 0.1-50 atm.^{2,3} | 0.2-50² |
| | 10-232⁴ | - | 0.05-20⁵ |
| | (27-204)⁴ | - | (0.1-10)⁵ |
| Paraffins to aromatics | 100-700 | 0-1000 psig, 0-6.89 MPa gauge | 0.5-40⁵ |
| Condensation of alcohols | 260-538 | 0.5-1000 psig, 0.00345-6.89 MPa gauge | 0.5-50⁵ |
| Isomerization | 93-538 | 50-1000 psig, 0.345-6.89 MPa gauge | 1-10 |
| | (204-315) | | (1-4) |
| Xylene isomerization | 260-593² | 0.5-50 atm.² | 0.1-100⁵ |
| | (315-566)² | (1-5 atm)² | (0.5-50)⁵ |
| | 38-371⁴ | 1-200 atm.⁴ | 0.5-50 |

| | | | |
|---|---|---|---|
| ¹Several hundred atmospheres (1 atm = 101 kPa) ² Gas phase reaction ³ Hydrocarbon partial pressure ⁴ Liquid phase reaction ⁵ WHSV | | | |

Other reaction conditions and parameters are provided below.

### Hydrocracking

Using a catalyst which comprises SSZ-58, preferably predominantly in the hydrogen form, and a hydrogenation promoter, heavy petroleum residual feedstocks, cyclic stocks and other hydrocrackate charge stocks can be hydrocracked using the process conditions and catalyst components disclosed in the aforementioned U.S. Patent No. 4,910,006 and U.S. Patent No. 5,316,753.

The hydrocracking catalysts contain an effective amount of at least one hydrogenation component of the type commonly employed in hydrocracking catalysts. The hydrogenation component is generally selected from the group of hydrogenation catalysts consisting of one or more metals of Group VIB and Group VIII, including the salts, complexes and solutions containing such. The hydrogenation catalyst is preferably selected from the group of metals, salts and complexes thereof of the group consisting of at least one of platinum, palladium, rhodium, iridium, ruthenium and mixtures thereof or the group consisting of at least one of nickel, molybdenum, cobalt, tungsten, titanium, chromium and mixtures thereof. Reference to the catalytically active metal or metals is intended to encompass such metal or metals in the elemental state or in some form such as an oxide, sulfide, halide, carboxylate and the like. The hydrogenation catalyst is present in an effective amount to provide the hydrogenation function of the hydrocracking catalyst, and preferably in the range of from 0.05 to 25% by weight.

### Dewaxing

SSZ-58, preferably predominantly in the hydrogen form, can be used to dewax hydrocarbonaceous feeds by selectively removing straight chain paraffins. Typically, the viscosity index of the dewaxed product is improved (compared to the waxy feed) when the waxy feed is contacted with SSZ-58 under isomerization dewaxing conditions.

The catalytic dewaxing conditions are dependent in large measure on the feed used and upon the desired pour point. Hydrogen is preferably present in the reaction zone during the catalytic dewaxing process. The hydrogen to feed ratio is typically between 0.089 to 5.34 SLM/liter (about 500 and about 30,000 SCF/bbl (standard cubic feet per barrel) preferably about 0.178 to 3.56 SCM/liter (1000 to about 20,000 SCF/bbl). Generally, hydrogen will be separated from the product and recycled to the reaction zone. Typical feedstocks include light gas oil, heavy gas oils and reduced crudes boiling above about 177°C (350°F).

A typical dewaxing process is the catalytic dewaxing of a hydrocarbon oil feedstock boiling above about (350°F) 177°C and containing straight chain and slightly branched chain hydrocarbons by contacting the hydrocarbon oil feedstock in the presence of added hydrogen gas at a hydrogen pressure of about 0.103-20.7 MPa (15-3000 psi) with a catalyst comprising SSZ-58 and at least one Group VIII metal.

The SSZ-58 hydrodewaxing catalyst may optionally contain a hydrogenation component of the type commonly employed in dewaxing catalysts. See the aforementioned U.S. Patent No. 4,910,006 and U.S. Patent No. 5,316,753 for examples of these hydrogenation components.

The hydrogenation component is present in an effective amount to provide an effective hydrodewaxing and hydroisomerization catalyst preferably in the range of from about 0.05 to 5% by weight. The catalyst may be run in such a mode to increase isodewaxing at the expense of cracking reactions.

The feed may be hydrocracked, followed by dewaxing. This type of two stage process and typical hydrocracking conditions are described in U.S. Patent No. 4,921;594, issued May 1,1990 to Miller.

SSZ-58 may also be utilized as a dewaxing catalyst in the form of a layered catalyst. That is, the catalyst comprises a first layer comprising zeolite SSZ-58 and at least one Group VIII metal, and a second layer comprising an aluminosilicate zeolite which is more shape selective than zeolite SSZ-58. The use of layered catalysts is disclosed in U.S. Patent No. 5,149,421, issued September 22,1992 to Miller The layering may also include a bed of SSZ-58 layered with a non-zeolitic component designed for either hydrocracking or hydrofinishing.

SSZ-58 may also be used to dewax raffinates, including bright stock, under conditions such as those disclosed in U. S. Patent No. 4,181,598, issued January 1, 1980 to Gillespie et al..

It is often desirable to use mild hydrogenation (sometimes referred to as hydrofinishing) to produce more stable dewaxed products. The hydrofinishing step can be performed either before or after the dewaxing step, and preferably after. Hydrofinishing is typically conducted at temperatures ranging from about 190°C to about 340°C at pressures from about 2.76 MPa gauge (400 psig) to about 20.7 MPa gauge (3000 psig) at space velocities (LHSV) between about 0.1 and 20 and a hydrogen recycle rate of about 0.071 to 0.267 SCL (standard cubic metres)/litre (400 to 1500 SCF/bbl). The hydrogenation catalyst employed must be active enough not only to hydrogenate the olefins, diolefins and color bodies which may be present, but also to reduce the aromatic content. Suitable hydrogenation catalyst are disclosed in U. S. Patent No. 4,921,594, issued May 1, 1990 to Miller. The hydrofinishing step is beneficial in preparing an acceptably stable product (e.g., a lubricating oil) since dewaxed products prepared from hydrocracked stocks tend to be unstable to air and light and tend to form sludges spontaneously and quickly.

Lube oil may be prepared using SSZ-58. For example, a C₂₀₊ lube oil may be made by isomerizing a C₂₀₊ olefin feed over a catalyst comprising SSZ-58 in the hydrogen form and at least one Group VIII metal. Alternatively, the lubricating oil may be made by hydrocracking in a hydrocracking zone a hydrocarbonaceous feedstock to obtain an effluent comprising a hydrocracked oil, and catalytically dewaxing the effluent at a temperature of at least about 204°C (400°F) and at a pressure of from about 0.103 MPa gauge (15 psig) to about 20.7 MPa gauge (3000 psig) in the presence of added hydrogen gas with a catalyst comprising SSZ-58 in the hydrogen form and at least one Group VIII metal.

### Aromatics Formation

SSZ-58 can be used to convert light straight run naphthas and similar mixtures to highly aromatic mixtures. Thus, normal and slightly branched chained hydrocarbons, preferably having a boiling range above about 40°C and less than about 200°C, can be converted to products having a substantial higher octane aromatics content by contacting the hydrocarbon feed with a catalyst comprising SSZ-58. It is also possible to convert heavier feeds into BTX or naphthalene derivatives of value using a catalyst comprising SSZ-58.

The conversion catalyst preferably contains a Group VIII metal compound to have sufficient activity for commercial use. By Group VIII metal compound as used herein is meant the metal itself or a compound thereof. The Group VIII noble metals and their compounds, platinum, palladium, and iridium, or combinations thereof can be used. Rhenium or tin or a mixture thereof may also be used in conjunction with the Group VIII metal compound and preferably a noble metal compound. The most preferred metal is platinum. The amount of Group VIII metal present in the conversion catalyst should be within the normal range of use in reforming catalysts, from about 0.05 to 2.0 weight percent, preferably 0.2 to 0.8 weight percent.

It is critical to the selective production of aromatics in useful quantities that the conversion catalyst be substantially free of acidity, for example, by neutralizing the zeolite with a basic metal, e.g., alkali metal, compound. Methods for rendering the catalyst free of acidity are known in the art. See the aforementioned U.S. Patent No. 4,910,006 and U.S. Patent No. 5,316,753 for a description of such methods.

The preferred alkali metals are sodium, potassium, rubidium and cesium. The zeolite itself can be substantially free of acidity only at very high silica:alumina mole ratios.

### Catalytic Cracking

Hydrocarbon cracking stocks can be catalytically cracked in the absence of hydrogen using SSZ-58, preferably predominantly in the hydrogen form.

When SSZ-58 is used as a catalytic cracking catalyst in the absence of hydrogen, the catalyst may be employed in conjunction with traditional cracking catalysts, e.g., any aluminosilicate heretofore employed as a component in cracking catalysts. Typically, these are large pore, crystalline aluminosilicates. Examples of these traditional cracking catalysts are disclosed in the aforementioned U.S. Patent No. 4,910,006 and U.S. Patent No 5,316,753. When a traditional cracking catalyst (TC) component is employed, the relative weight ratio of the TC to the SSZ-58 is generally between about 1:10 and about 500:1, desirably between about 1:10 and about 200:1, preferably between about 1:2 and about 50:1, and most preferably is between about 1:1 and about 20:1. The novel zeolite and/or the traditional cracking component may be further ion exchanged with rare earth ions to modify selectivity.

The cracking catalysts are typically employed with an inorganic oxide matrix component. See the aforementioned U.S. Patent No. 4,910,006 and U.S. Patent No. 5,316,753 for examples of such matrix components.

### Isomerization

The present catalyst is highly active and highly selective for isomerizing C₄ to C₇ hydrocarbons. The activity means that the catalyst can operate at relatively low temperature which thermodynamically favors highly branched paraffins. Consequently, the catalyst can produce a high octane product. The high selectivity means that a relatively high liquid yield can be achieved when the catalyst is run at a high octane.

The present process comprises contacting the isomerization catalyst, i.e., a catalyst comprising SSZ-58 in the hydrogen form, with a hydrocarbon feed under isomerization conditions. The feed is preferably a light straight run fraction, boiling within the range of -1.1°C (30°F) to 121°C (250°F) and preferably from (60°F) (16°C) to 200°F (93°C). Preferably, the hydrocarbon feed for the process comprises a substantial amount of C₄ to C₇ normal and slightly branched low octane hydrocarbons, more preferably C₅ and C₆ hydrocarbons.

It is preferable to carry out the isomerization reaction in the presence of hydrogen. Preferably, hydrogen is added to give a hydrogen to hydrocarbon ratio (H₂/HC) of between 0.5 and 10 H₂/HC, more preferably between 1 and 8 H₂/HC. See the aforementioned U.S. Patent No. 4,910,006 and U.S. Patent No. 5,316,753 for a further discussion of isomerization process conditions.

A low sulfur feed is especially preferred in the present process. The feed preferably contains less than 10 ppm, more preferably less than 1 ppm, and most preferably less than 0.1 ppm sulfur. In the case of a feed which is not already low in sulfur, acceptable levels can be reached by hydrogenating the feed in a presaturation zone with a hydrogenating catalyst which is resistant to sulfur poisoning. See the aforementioned U.S. Patent No. 4,910,006 and U.S. Patent No. 5,316,753 for a further discussion of this hydrodesulfurization process.

It is preferable to limit the nitrogen level and the water content of the feed. Catalysts and processes which are suitable for these purposes are known to those skilled in the art.

After a period of operation, the catalyst can become deactivated by sulfur or coke. See the aforementioned U.S. Patent No. 4,910,006 and U.S. Patent No. 5,316,753 for a further discussion of methods of removing this sulfur and coke, and of regenerating the catalyst.

The conversion catalyst preferably contains a Group VIII metal compound to have sufficient activity for commercial use. By Group VIII metal compound as used herein is meant the metal itself or a compound thereof. The Group VIII noble metals and their compounds, platinum, palladium, and iridium, or combinations thereof can be used. Rhenium and tin may also be used in conjunction with the noble metal. The most preferred metal is platinum. The amount of Group VIII metal present in the conversion catalyst should be within the normal range of use in isomerizing catalysts, from about 0.05 to 2.0 weight percent, preferably 0.2 to 0.8 weight percent.

### Alkylation and Transalkylation

SSZ-58 can be used in a process for the alkylation or transalkylation of an aromatic hydrocarbon. The process comprises contacting the aromatic hydrocarbon with a C₂ to C₁₆ olefin alkylating agent or a polyalkyl aromatic hydrocarbon transalkylating agent, under at least partial liquid phase conditions, and in the presence of a catalyst comprising SSZ-58.

SSZ-58 can also be used for removing benzene from gasoline by alkylating the benzene as described above and removing the alkylated product from the gasoline.

For high catalytic activity, the SSZ-58 zeolite should be predominantly in its hydrogen ion form. It is preferred that, after calcination, at least 80% of the cation sites are occupied by hydrogen ions and/or rare earth ions.

Examples of suitable aromatic hydrocarbon feedstocks which may be alkylated or transalkylated by the process of the invention include aromatic compounds such as benzene, toluene and xylene. The preferred aromatic hydrocarbon is benzene. There may be occasions where naphthalene or naphthalene derivatives, such as dimethylnaphthalene, may be desirable. Mixtures of aromatic hydrocarbons may also be employed.

Suitable olefins for the alkylation of the aromatic hydrocarbon are those containing 2 to 20, preferably 2 to 4, carbon atoms, such as ethylene, propylene, butene-1, trans-buten-2 and cis-butene-2, or mixtures thereof. There may be instances where pentenes are desirable. The preferred olefins are ethylene and propylene. Longer chain alpha olefins may be used as well.

When transalkylation is desired, the transalkylating agent is a polyalkyl aromatic hydrocarbon containing two or more alkyl groups that each may have from 2 to about 4 carbon atoms. For example, suitable polyalkyl aromatic hydrocarbons include di-, tri- and tetra-alkyl aromatic hydrocarbons, such as diethylbenzene, triethylbenzene, diethylmethylbenzene (diethyltoluene), di-isopropylbenzene, di-isopropyltoluene, dibutylbenzene, and the like. Preferred polyalkyl aromatic hydrocarbons are the dialkyl benzenes. A particularly preferred polyalkyl aromatic hydrocarbon is di-isopropylbenzene.

When alkylation is the process conducted, reaction conditions are as follows. The aromatic hydrocarbon feed should be present in stoichiometric excess. It is preferred that molar ratio of aromatics to olefins be greater than four-to-one to prevent rapid catalyst fouling. The reaction temperature may range from 38°C (100°F) to 316°C (600°F), preferably 121°C (250°F) to 232°C (450°C). The reaction pressure should be sufficient to maintain at least a partial liquid phase, in order to retard catalyst fouling. This is typically 50 psig (0.345 MPa gauge to 1000 psig (6.39 MPa gauge) depending on the feedstock and reaction temperature. Contact time may range from 10 seconds to 10 hours, but is usually from 5 minutes to an hour. The weight hourly space velocity (WHSV), in terms of grams (pounds) of aromatic hydrocarbon and olefin per gram (pound) of catalyst per hour, is generally within the range of about 0.5 to 50.

When transalkylation is the process conducted, the molar ratio of aromatic hydrocarbon will generally range from about 1:1 to 25:1, and preferably from about 2:1 to 20:1. The reaction temperature may range from about 38°C (100°F) to 316°C (600°F) but it is preferably about 121°C (250°F) to 232°C (450°F). The reaction pressure should be sufficient to maintain at least a partial liquid phase typically in the range of about 0.345 MPa gauge (50 psig) to 6.89 MPa gauge (1000 psig), preferably (300 psig 2.07 MPa gauge to 0.17 MPa gauge to 600 psig). The weight hourly space velocity will range from about 0.1 to 10. U.S. Patent No. 5,082,990 issued on January 21, 1992 to Hsieh, et al. describes such processes.

### Conversion of Paraffins to Aromatics

SSZ-58 can be used to convert light gas C₂-C₆ paraffins to higher molecular weight hydrocarbons including aromatic compounds. Preferably, the zeolite will contain a catalyst metal or metal oxide wherein said metal is selected from the group consisting of Groups IB, IIB, VIII and IIIA of the Periodic Table. Preferably, the metal is gallium, niobium, indium or zinc in the range of from about 0.05 to 5% by weight.

### Xylene Isomerization

SSZ-58 may also be useful in a process for isomerizing one or more xylene isomers in a C₈ aromatic feed to obtain ortho-, meta-, and para-xylene in a ratio approaching the equilibrium value. In particular, xylene isomerization is used in conjunction with a separate process to manufacture para-xylene. For example, a portion of the para-xylene in a mixed C₈ aromatics stream may be recovered by crystallization and centrifugation. The mother liquor from the crystallizer is then reacted under xylene isomerization conditions to restore ortho-, meta- and para-xylenes to a near equilibrium ratio. At the same time, part of the ethylbenzene in the mother liquor is converted to xylenes or to products which are easily separated by filtration. The isomerate is blended with fresh feed and the combined stream is distilled to remove heavy and light byproducts. The resultant C₈ aromatics stream is then sent to the crystallizer to repeat the cycle.

Optionally, isomerization in the vapor phase is conducted in the presence of 3.0 to 30.0 moles of hydrogen per mole of alkylbenzene (e.g., ethylbenzene). If hydrogen is used, the catalyst should comprise about 0.1 to 2.0 wt.% of a hydrogenation/dehydrogenation component selected from Group VIII (of the Periodic Table) metal component, especially platinum or nickel. By Group VIII metal component is meant the metals and their compounds such as oxides and sulfides.

Optionally, the isomerization feed may contain 10 to 90 wt.% of a diluent such as toluene, trimethylbenzene, naphthenes or paraffins.

### Oligomerization

It is expected that SSZ-58 can also be used to oligomerize straight and branched chain olefins having from about 2 to 21 and preferably 2-5 carbon atoms. The oligomers which are the products of the process are medium to heavy olefins which are useful for both fuels, i.e., gasoline or a gasoline blending stock and chemicals.

The oligomerization process comprises contacting the olefin feedstock in the gaseous or liquid phase with a catalyst comprising SSZ-58.

The zeolite can have the original cations associated therewith replaced by a wide variety of other cations according to techniques well known in the art. Typical cations would include hydrogen, ammonium and metal cations including mixtures of the same. Of the replacing metallic cations, particular preference is given to cations of metals such as rare earth metals, manganese, calcium, as well as metals of Group II of the Periodic Table, e.g., zinc, and Group VIII of the Periodic Table, e.g., nickel. One of the prime requisites is that the zeolite have a fairly low aromatization activity, i.e., in which the amount of aromatics produced is not more than about 20% by weight. This is accomplished by using a zeolite with controlled acid activity [alpha value] of from about 0.1 to about 120, preferably from about 0.1 to about 100, as measured by its ability to crack n-hexane.

Alpha values are defined by a standard test known in the art, e.g., as shown in U.S. Patent No. 3,960,978 issued on June 1, 1976 to Givens et al.. If required, such zeolites may be obtained by steaming, by use in a conversion process or by any other method which may occur to one skilled in this art.

### Condensation of Alcohols

SSZ-58 can be used to condense lower aliphatic alcohols having 1 to 10 carbon atoms to a gasoline boiling point hydrocarbon product comprising mixed aliphatic and aromatic hydrocarbon. The process disclosed in U.S. Patent No. 3,894,107, issued July 8, 1975 to Butter et al., describes the process conditions used in this process.

The catalyst may be in the hydrogen form or may be base exchanged or impregnated to contain ammonium or a metal cation complement, preferably in the range of from about 0.05 to 5% by weight. The metal cations that may be present include any of the metals of the Groups through VIII of the Periodic Table. However, in the case of Group IA metals, the cation content should in no case be so large as to effectively inactivate the catalyst, nor should the exchange be such as to eliminate all acidity. There may be other processes involving treatment of oxygenated substrates where a basic catalyst is desired.

### Methane Upgrading

Higher molecular weight hydrocarbons can be formed from lower molecular weight hydrocarbons by contacting the lower molecular weight hydrocarbon with a catalyst comprising SSZ-58 and a metal or metal compound capable of converting the lower molecular weight hydrocarbon to a higher molecular weight hydrocarbon. Examples of such reactions include the conversion of methane to C₂₊ hydrocarbons such as ethylene or benzene or both. Examples of useful metals and metal compounds include lanthanide and or actinide metals or metal compounds.

These reactions, the metals or metal compounds employed and the conditions under which they can be run are disclosed in U.S. Patents No. 4,734,537, issued March 29, 1988 to Devries et al.; 4,939,311, issued July 3, 1990 to Washecheck et al.; 4,962,261, issued October 9, 1990 to Abrevaya et al.; 5,095,161, issued March 10, 1992 to Abrevaya et al.; 5,105,044, issued April 14,1992 to Han et al.; 5,105,046, issued April 14, 1992 to Washecheck; 5,238,898, issued August 24, 1993 to Han et al.; 5,321,185, issued June 14, 1994 to van der Vaart; and 5,336,825, issued August 9, 1994 to Choudhary et al..

SSZ-58 may be used for the catalytic reduction of the oxides of nitrogen in a gas stream. Typically, the gas stream also contains oxygen, often a stoichiometric excess thereof. Also, the SSZ-58 may contain a metal or metal ions within or on it which are capable of catalyzing the reduction of the nitrogen oxides. Examples of such metals or metal ions include copper, cobalt and mixtures thereof.

One example of such a process for the catalytic reduction of oxides of nitrogen in the presence of a zeolite is disclosed in U.S. Patent No. 4,297,328, issued October 27, 1981 to Ritscher et al.. There, the catalytic process is the combustion of carbon monoxide and hydrocarbons and the catalytic reduction of the oxides of nitrogen contained in a gas stream, such as the exhaust gas from an internal combustion engine. The zeolite used is metal ion-exchanged, doped or loaded sufficiently so as to provide an effective amount of catalytic copper metal or copper ions within or on the zeolite. In addition, the process is conducted in an excess of oxidant, e.g., oxygen.

### EXAMPLES

The following examples demonstrate but do not limit the present invention. The templating agent indicated Table C below is used in these examples.

**Table C**

| |
|---|
| 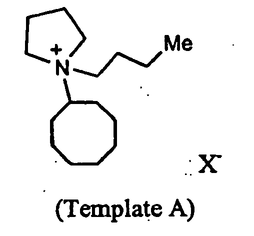 |
| 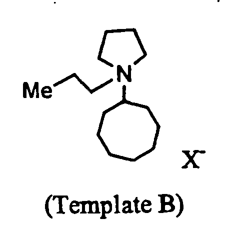 |

The anion (X⁻) associated with the cation may be any anion which is not detrimental to the formation of the zeolite. Representative anions include halogen, e.g., fluoride, chloride, bromide and iodide, hydroxide, acetate, sulfate, tetrafluoroborate, carboxylate, and the like. Hydroxide is the most preferred anion.

### Example 1

### Synthesis of N-butyl-N-cyclooctylpyrrolidinium hydroxide (Template A)

### I. Synthesis of N-cyclooctylpyrrolidine

A three-neck 3000 ml. flask was charged with 75 gm. (1.05 moles) of pyrrolidine, 51 g. cyclooctanone (0.4 mole) and 80 ml. anhydrous hexane. To the resulting solution, 80 gm. (0.8 mole) of anhydrous magnesium sulfate was added and the mixture was mechanically stirred and heated at reflux (the reaction was monitored by NMR analysis) for 108 hours. The reaction mixture was filtered through a fritted glass funnel. The filtrate was concentrated at reduced pressure on a rotary evaporator to give 70.5 g. of a clear (yellow-tinted) oily substance. ¹H-NMR and ¹³C-NMR spectra were acceptable for the desired product, 1-(1-pyrrolino)cyclooctene. Saturation of the 1-(1-pyrrolino)cyclooctene to give N-cyclooctylpyrrolidine was accomplished in 98% yield by catalytic hydrogenation in ethanol at a 0.379 MPa (55 psi) pressure of hydrogen gas in the presence of 10% Pd on activated carbon.

### II. Quaternization (synthesis of N-butyl-N-cyclooctylpyrrolidinium iodide)

To a solution of 60 g. (0.33 mole) of N-cyclooctyl pyrrolidine in 600 ml. anhydrous methanol, 150 g. (0.825 mole) of butyl iodide was added. The reaction mixture was refluxed while stirring for four days. Then an additional equivalent of butyl iodide and one equivalent (33 g., 0.33 mole) of potassium bicarbonate were added and the mixture was stirred at refluxing temperature for an additional 36 hours. The reaction mixture was concentrated at reduced pressure on a rotary evaporator to give an off-white colored solid material. The solids were rinsed several times with chloroform and filtered after each rinse. All the chloroform rinses were combined and concentrated to give a white powder whose NMR data were acceptable for the desired quaternary ammonium iodide salt. The reaction afforded 109 g. (90% yield) of N-butyl-N-cyclooctylpyrrolidinium iodide. The iodide salt was purified by recrystallization by completely dissolving the iodide salt in acetone, and then precipitating by the addition of ethyl ether to the acetone solution. This procedure gave 98 gms. of white powder with very clean ¹H and ¹³C-NRM spectra.

### III. Ion Exchange (synthesis of N-butyl-N-cyclooctylpyrrolidinium hydroxide)

N-butyl-N-cyclooctylpyrrolidinium iodide salt (95 g., 0.26 mole) was dissolved in 300 ml. water in a 1000 ml. plastic bottle. To the solution, 300 g. of Ion Exchange Resin OH (BIO RAD^{®} AG1-X8) was added and the mixture was stirred at room temperature overnight. The mixture was filtered and the solids were rinsed with an additional 250 ml. of water. The original mixture was filtered and the rinse were combined and a small amount was titrated with 0.1N HCl to indicate the presence of 0.24 mol hydroxide (0.24 mol N-butyl-N-cyclooctylpyrrolidinium hydroxide) in the solution.

The synthetic procedure described above is depicted below.

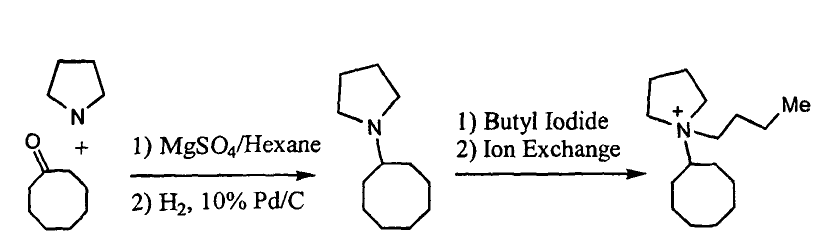

In a manner similar to that of Example 1, N-propyl-cyclooctylpyrrolidinium cation (Template B) can be prepared.

### Example 2

### Preparation of Borosilicate SSZ-58

A 23 ml Teflon liner was charged with 6.9 g. of 0.435M aqueous solution of N-butyl-N-cyclooctylpyrrolidinium hydroxide (3 mmol, Template A), 1.2 g. of 1M aqueous solution of NaOH (1.2 mmol NaOH) and 3.9 g. of deionized water. To the resulting mixture, 0.06 g. of sodium borate decahydrate (0.157 mmol of sodium borate decahydrate, about 0.315 mmol B₂O₃) was added and stirred until completely dissolved. Then 0.9 g. of Cabosil-M-5 fumed SiO₂ (about 14.7 mmol SiO₂) was added to the solution and the mixture was thoroughly stirred. The resulting gel was capped off and placed in a Parr bomb steel reactor and heated in an oven at 160° C. while rotating at 43 rpm. The reaction was monitored by checking the gel's pH, and by looking for crystal formation using Scanning Electron Microscopy (SEM) at six day intervals. The reaction was completed after heating for 12 days at the conditions described above. Once the crystallization was complete, the starting reaction gel turned to a mixture comprising a clear liquid layer with solids (powder) that settled to the bottom. The mixture was filtered through a fritted glass funnel. The collected solids were thoroughly washed with water and then rinsed with acetone (10 ml.) to remove any organic residues. The solids were allowed to air-dry overnight and then they were oven-dried at 120°C for one hour. The reaction afforded 0.78 g. of a very fine powder. SEM showed the presence of only one crystalline phase. The X-ray analysis of the powder indicated that the material was SSZ-58.

### Examples 3-16

### Synthesis of Borosilicate SSZ-58

The synthesis of Example 2 was repeated keeping the amount of NaOH, water and Cab-O-Sil M5 the same while varying the amount of Na₂B₄O₇ 10H₂O. The SiO₂/OH mole ratio was 3.5, the H₂O/SiO₂mole ratio was 45 and the SiO₂/B₂O₃ and SiO₂/Na mole ratios were as indicated in the table below. The reactions were carried out at 160° C. and 43 rpm.

| Example No. | SiO₂/B₂O₃ | SiO₂/Na | Days | Products |
|---|---|---|---|---|
| 3 | 280 | 11.74 | 12 | SSZ-58 |
| 4 | 140 | 11.26 | 12 | SSZ-58 |
| 5 | 93.6 | 10.83 | 12 | SSZ-58 |
| 6 | 70 | 10.42 | 12 | SSZ-58 |
| 7 | 56 | 10.05 | 12 | SSZ-58 |
| 8 | 46.3 | 9.7 | 12 | SSZ-58 |
| 9 | 40 | 9.38 | 12 | SSZ-58 |
| 10 | 35 | 9.07 | 12 | SSZ-58 |
| 11 | 31 | 8.8 | 18 | SSZ-58 |
| 12 | 28 | 8.52 | 18 | SSZ-58 + layered mat'l |
| 13 | 25.5 | 8.27 | 18 | SSZ-58 + layered mat'l |
| 14 | 23.3 | 8.03 | 18 | SSZ-58 (major) + layered mat'l (minor |
| 15 | 21.55 | 7.81 | 18 | SSZ-58 (major) + layered mat'l (minor) |
| 16 | 18.67 | 7.4 | 21 | SSZ-58 + layered mat'1 (minor) |

### Example 17

### Synthesis of Aluminosilicate SSZ-58

A 23 ml. Teflon liner was charged with 5.2 g. of 0.43 5M aqueous solution of N-butyl-N-cyclooctylpyrrolidinium hydroxide (2.25 mmol Template A), 1.5 g. of 1M NaOH aqueous solution (1.5 mmol NaOH) and 0.75 g. of deionized water. To the resulting solution, 0.25 g. of sodium-Y zeolite (Union Carbide LZ-Y52: SiO₂/Al₂O₃ = 5) and 0.80 g. of Cabosil M-5 fumed SiO₂ (about 13 mmol SiO₂) was added, consecutively. The resulting mixture was thoroughly stirred and the resulting gel was capped off and placed in a Parr bomb steel reactor and heated in an oven at 160° C. while rotating at 43 rpm. The reaction was monitored by checking the gel's pH, and by looking for crystal formation using SEM at six day intervals. The reaction was completed after heating at the conditions described above for six days. The completed reaction mixture appeared as a colorless liquid with fine white solid settled to the bottom of the Teflon liner. The mixture was filtered through a fritted glass funnel, and the obtained white solids were washed generously with water and then rinsed with a small amount of acetone and allowed to air-dry overnight. The solids were further dried in an oven at 120° C. for one hour. The reaction yielded 0.81 g. of SSZ-58.

### Examples 18-32

### Synthesis of Aluminosilicate SSZ-58

The synthesis of Example 17 was repeated using LZ-Y52 as the aluminum source and Cab-O-Sil M5 as the SiO₂ source. The SiO₂/OH mole ratio was 8.7, the H₂O/SiO₂mole ratio was 28 and the SiO₂/Al₂O₃ and SiO₂/Na mole ratios were as indicated in the table below. The reactions were carried out at 160° C. and 43 rpm.

| Example No. | SiO₂/Al₂O₃ | SiO₂/Na | Products |
|---|---|---|---|
| 18 | 317 | 8.4 | SSZ-58+TraceLZ-Y52 |
| 19 | 158.5 | 8.1 | SSZ-58 + Trace LZ-Y52 |
| 20 | 107.5 | 7.78 | SSZ-58+Trace LZ-Y52 |
| 21 | 82.5 | 7.5 | SSZ-58 |
| 22 | 66.9 | 7.3 | SSZ-58 |
| 23 | 56.5 | 7.1 | SSZ-58 |
| 24 | 49 | 6.9 | SSZ-58 |
| 25 | 43.5 | 6.7 | SSZ-58 |
| 26 | 39 | 6.6 | SSZ-58 + trace LZ-Y52 |
| 27 | 35.8 | 6.4 | SSZ-58 + trace LZ-Y52 |
| 28 | 33 | 6.26 | SSZ-58 (mostly) + LZ-Y52 |
| 29 | 30.8 | 6.16 | SSZ-58 (mostly) + LZ-Y52 |
| 30 | 26.3 | 5.85 | SSZ-58 (major) LZ-Y52 (minor) |
| 31 | 23.8 | 5.66 | SSZ-58 (major) LZ-Y52 (minor) |
| 32 | 20 | 5.32 | SSZ-58 (major) LZ-Y52 (minor) |

### Example 33

### Synthesis of All-Silica SSZ-58

A 23ml. Teflon liner was charged with 6.9 g. of 0.435M aqueous solution of N-butyl-N-cyclooctylpyrrolidinium hydroxide (3 mmol Template A), 1.2 g. of 1M NaOH aqueous solution (1.2 mmol NaOH) and 3.9 g. of deionized water. To the resulting solution, 0.9 g. of Cabosil M-5 fumed SiO₂ (about 14.7 mmol SiO₂) was added and the mixture was thoroughly stirred. The resulting mixture was thoroughly stirred and the resulting gel was capped off and placed in a Parr bomb steel reactor and heated in an oven at 160° C. while rotating at 43 rpm. The reaction was monitored by checking the gel's pH, and by looking for crystal formation using SEM at six day intervals. The reaction was completed after heating at the conditions described above for 18 days. The completed reaction mixture appeared as a colorless liquid with solids (powder) settled to the bottom of the Teflon liner. The mixture was filtered through a fritted glass funnel. The collected solids were thoroughly washed with water and then rinsed with acetone (10 ml.) to remove any organic residues. The solids were allowed to air-dry overnight and then dried in an oven at 120° C. for one hour. The reaction yielded 0.73 g. of pure SSZ-58

### Example 34

### Seeded Synthesis of Borosilicate SSZ-58

A 23 ml Teflon liner is charged with 6.9 g of 0.435M aqueous solution of N-butyl-N-cyclooctylpyrrolidinium hydroxide (3 mmol template), 1.2 g of 1M aqueous solution of NaOH (1.2 mmol NaOH) and 3.9 g of de-ionized water. To this mixture, 0.06 g of sodium borate decahydrate (0.157 mmol of Na₂B₄O₇.10H₂O; ~0.315 mmol B₂O₃) is added and stirred until completely dissolved. Then, 0.9 g of CABOSIL-M-5 (~14.7 mmol SiO₂) and 0.04 g of SSZ-58 (the product of Example 1) is added to the solution and the mixture is thoroughly stirred. The resulting gel is capped off and placed in a Parr bomb steel reactor and heated in an oven at 160°C while rotating at 43 rpm. The reaction is monitored by checking the gel's pH, and by looking for crystal formation using Scanning Electron Microscopy (SEM). The reaction is completed after heating for 5 days at the conditions described above. Once the crystallization is complete, the starting reaction gel turns to a mixture comprising of a clear liquid layer with solids (powder) that settled to the bottom. The mixture is filtered through a fitted-glass funnel. The collected solids are thoroughly washed with water and, then, rinsed with acetone (10 ml) to remove any organic residues. The solids are allowed to air-dry over night and, then, dried in an oven at 120 °C for one hour. The reaction affords 0.85 gram of a very fine powder. SEM shows the presence of only one crystalline phase. The X-ray pattern of the powder is identical to the XRD pattern of the product of Example 1.

### Example 35

### Calcination of SSZ-58

The material from Example 2 is calcined in the following manner. A thin bed of material is heated in a muffle furnace from room temperature to 120°C at a rate of 1°C per minute and held at 120°C for three hours. The temperature is then ramped up to 540°C at the same rate and held at this temperature for 5 hours, after which it is increased to 594°C and held there for another 5 hours. A 50/50 mixture of air and nitrogen is passed over the zeolite at a rate of 566 l/min (20 standard cubic feet per minute) during heating. The product had the X-ray diffraction data Table IIA above.

### Example 36

### NH₄ Exchange

Ion exchange of calcined SSZ-58 material (prepared in Example 35) is performed using NH₄NO₃ to convert the zeolite from its Na⁺ form to the NH₄⁺ form, and, ultimately, the H⁺ form. Typically, the same mass of NH₄NO₃ as zeolite is slurried in water at a ratio of 25-50:1 water to zeolite. The exchange solution is heated at 95°C for 2 hours and then filtered. This procedure can be repeated up to three times. Following the final exchange, the zeolite is washed several times with water and dried. This NH₄⁺ form of SSZ-58 can then be converted to the H⁺ form by calcination (as described in Example 35) to 540°C.

### Example 37

### Constraint Index Determination

The hydrogen form of the zeolite of Example 17 (after treatment according to Examples 34 and 35) is pelletized at 13790-20684 kN/m² (2-3 KPSI), crushed and meshed to 0.422-0.853 mm (20-40), and then > 0.50 gram is calcined at about 540°C in air for four hours and cooled in a desiccator. 0.50 Gram is packed into 9.5mm a (3/8 inch) stainless steel tube with alundum on both sides of the zeolite bed. A Lindburg furnace is used to heat the reactor tube. Helium is introduced into the reactor tube at 10 ml/min. and at atmospheric pressure. The reactor is heated to about 315°C, and a 50/50 (w/w) feed of n-hexane and 3-methylpentane is introduced into the reactor at a rate of 8 µl/min. Feed delivery is made via an ISCO pump. Direct sampling into a gas chromatograph begins after 10 minutes of feed introduction. The Constraint Index value is calculated from the gas chromatographic data using methods known in the art, and is found to be 0.57. At 315°C and 10 minutes on-stream, feed conversion was 37%.

It can be seen that SSZ-58 has very high cracking activity, indicative of strongly acidic sites. The low value of the Constraint Index and the fouling rate of SSZ-58 are typical of a large pore zeolite. In addition, the low fouling rate indicates that this catalyst has a good stability.

### Example 38

### n-Hexadecane Cracking

The product of Example 17 is treated as in Examples 34 and 35. Then a sample is slurried in water and the pH of the slurry adjusted to a pH of~10 with dilute ammonium hydroxide. To the slurry is added a solution of Pd(NH₃)₄(NO₃)₂ at a concentration which would provide 0.5 wt. % Pd with respect to the dry weight of the zeolite sample. This slurry is left to stand at room temperature for 72 hours. Then, the slurry is filtered through a fritted glass funnel, washed with de-ionized water, and dried at 120°C for two hours. The catalyst is then calcined slowly up to 482°C in air and held there for three hours.

The calcined catalyst is pelletized in a Carver Press and crushed to yield particles with a 0.422-0.853 mm (20/40 mesh) size range. mesh) size range. 0.5 g of the catalyst is packed into a 6.35 mm (¼") OD tubing reactor in a micro unit for n-hexadecane hydroconversion. Table III gives the run conditions and the products data for the hydrocracking test on n-hexadecane. After the catalyst is tested with n-hexadecane, it is titrated using a solution of butyl amine in hexane. The temperature is increased and the conversion and product data evaluated again under titrated conditions. The results shown in Table III show that SSZ-58 is an effective hydrocracking catalyst.

**Table III**

| Temperature | 279°C (534°F) | 306°C (582°F) |
|---|---|---|
| Time-on-Stream (hrs.) | 33.8-45.7 | 57.7-70.2 |
| WHSV | 1.55 | 1.55 |
| PSIG (1 PSIG = 6.89 kPa) | 1200 | 1200 |
| Titrated? | No | Yes |
| n-16, % Conversion | 97.7 | 99.4 |
| Hydrocracking Conversion, % | 70.1 | 79.6 |
| Isomerization Selectivity, % | 29.4 | 24.4 |
| Crack. Selectivity, % | 70.6 | 78.1 |
| C₄⁻, % | 8.4 | 8.6 |
| C₅/C₄ | 7.4 | 7.9 |
| C₅+C₆/C₅, % | 25.8 | 28.3 |
| DMB/MP | 0.04 | 0.04 |
| C₄-C₁₃ I/N | 1.64 | 2.1 |

### Example 39

### Nitrogen Adsorption

The product of Example 2 is treated as in Examples 34 and 35. Then it is subjected to a surface area and micropore volume analysis using N₂ as adsorbate and via the BET method. The BET area is 326 m²/g. The external surface area of the zeolite is 88 m²/gm and the micropore volume is 0.11 ml/g.

### Example 40

Using a procedure similar to that of Example 2, SSZ-58 is prepared using a N-propyl-cyclooctylpyrrolidinium cation (Template B) as the templating agent.

## Claims

1. A zeolite having a mole ratio greater than about 20 of an oxide of a first tetravalent element to an oxide of a second tetravalent element which is different from said first tetravalent element, trivalent element, pentavalent element or mixture thereof and having, after calcination, the X-ray diffraction lines of Table II below:
**TABLE II**
| Calcined SSZ-58 | | |
|---|---|---|
| 2 Theta (deg.)^{(a)} | d (Å / × 10⁻¹⁰)m | Relative Intensity |
| 7.1 | 12.4 | VS |
| 7.7 | 11.5 | M |
| 9.9 | 8.93 | M |
| 10.5 | 8.42 | M |
| 12.1 | 7.31 | W |
| 17.3 | 5.12 | W |
| 19.8 | 4.48 | M |
| 21.0 | 4.23 | S |
| 21.9 | 4.06 | M |
| 22.4 | 3.97 | S |
| | | |
|---|---|---|
| ^{(a)} ± 0.15 | | |

2. A zeolite according to claim 1 having a mole ratio greater than about 20 of an oxide selected from the group consisting of silicon oxide, germanium oxide and mixtures thereof to an oxide selected from aluminum oxide, gallium oxide, iron oxide, boron oxide, titanium oxide, indium oxide, vanadium oxide and mixtures thereof.

3. A zeolite according to Claim 2 wherein the oxides comprise silicon oxide and aluminum oxide.

4. A zeolite according to Claim 2 wherein the oxides comprise silicon oxide and boron oxide.

5. A zeolite according to Claim 1 wherein said zeolite is predominantly in the hydrogen form.

6. A zeolite according to Claim 1 wherein said zeolite is substantially free of acidity.

7. A zeolite according to claim 1 or 2 having a composition, as synthesized and in the anhydrous state, in terms of mole ratios as follows:
YO₂/W_{c}O_{d} >20
M_{2/n}/YO₂ 0.01 - 0.03
Q/YO₂ 0.02 - 0.05
wherein Y is silicon, germanium or a mixture thereof; W is aluminum, gallium, iron, boron, titanium, indium, vanadium or mixtures thereof; c is 1 or 2; d is 2 when c is 1 or d is 3 or 5 when c is 2; M is an alkali metal cation, alkaline earth metal cation or mixtures thereof; n is the valence of M; and Q is a N-butyl-N-cyclooctylpyrrolidinium cation or N-propyl-cyclooctylpyrrolidinium cation.

8. A zeolite according to Claim 7 wherein W is aluminum and Y is silicon.

9. A zeolite according to Claim 7 wherein W is boron and Y is silicon.

10. A zeolite according to Claim 7 wherein Q a N-butyl-cyclooctylpyrrolidinium cation.

11. A zeolite according to Claim 7 wherein Q is a N-propyl-cyclooctylpyrrolidinium cation.

12. A method of preparing the zeolite of claim 1 or 2 comprising contacting under crystallization conditions sources of said oxides and a templating agent comprising a N-butyl-N-cyclooctylpyrrolidinium cation or N-propyl-cyclooctylpyrrolidinium cation.

13. The method according to Claim 12 wherein the first tetravalent element is selected from the group consisting of silicon, germanium and combinations thereof.

14. The method according to Claim 12 wherein the second tetravalent element, trivalent element or pentavalent element is selected from the group consisting of aluminum, gallium, iron, boron, titanium, indium, vanadium and combinations thereof.

15. The method according to Claim 14 wherein the second tetravalent element or trivalent element is selected from the group consisting of aluminum, boron, titanium and combinations thereof.

16. The method according to Claim 15 wherein the first tetravalent element is silicon.

17. The method according to Claim 12 wherein the templating agent is a N-butylcyclooctylpyrrolidinium cation.

18. The method according to Claim 12 wherein the templating Agent is a N-propylcyclooctylpyrrolidinium cation.

19. A process for converting hydrocarbons comprising contacting a hydrocarbonaceous feed at hydrocarbon converting conditions with a catalyst comprising the zeolite according to claim 1 or 2.

20. The process of Claim 19 wherein the zeolite is predominantly in the hydrogen form.

21. The process of Claim 19 wherein the zeolite is substantially free of acidity.

22. The process of Claim 19 wherein the process is a hydrocracking process comprising contacting the catalyst with a hydrocarbon feedstock under hydrocracking conditions.

23. The process of Claim 22 wherein the zeolite is predominantly in the hydrogen form.

24. The process of Claim 19 wherein the process is a dewaxing process comprising contacting the catalyst with a hydrocarbon feedstock under dewaxing conditions.

25. The process of Claim 24 wherein the zeolite is predominantly in the hydrogen form.

26. The process of Claim 19 wherein the process is a process for improving the viscosity index of a dewaxed product of waxy hydrocarbon feeds comprising contacting the catalyst with a waxy hydrocarbon feed under isomerization dewaxing conditions.

27. The process of Claim 26 wherein the zeolite is predominantly in the hydrogen form.

28. The process of Claim 19 wherein the process is a process for producing a C₂₀₊ lube oil from a C₂₀₊ olefin feed comprising isomerizing said olefin feed under isomerization conditions over the catalyst.

29. The process of Claim 28 wherein the zeolite is predominantly in the hydrogen form.

30. The process of Claim 28 wherein the catalyst further comprises at least one Group VIII metal.

31. The process of Claim 19 wherein the process is a process for catalytically dewaxing a hydrocarbon oil feedstock boiling above about 177°C (350°F) and containing straight chain and slightly branched chain hydrocarbons comprising contacting said hydrocarbon oil feedstock in the presence of added hydrogen gas at a hydrogen pressure of about 15-3000 psi (0.10-20.68MPa) under dewaxing conditions with the catalyst.

32. The process of Claim 31 wherein the zeolite is predominantly in the hydrogen form.

33. The process of Claim 31 wherein the catalyst further comprises at least one Group VIII metal.

34. The process of Claim 31 wherein said catalyst comprises a layered catalyst comprising a first layer comprising the zeolite and at least one Group VIII metal, and a second layer comprising an aluminosilicate zeolite which is more shape selective than the zeolite of said first layer.

35. The process of Claim 19 wherein the process is a process for preparing a lubricating oil which comprises:
hydrocracking in a hydrocracking zone a hydrocarbonaceous feedstock to obtain an effluent comprising a hydrocracked oil; and
catalytically dewaxing said effluent comprising hydrocracked oil at a temperature of at least about (204°C) (400°F) and at a pressure of from about 0.103 MPa gauge (5 psig) to about (20.7 MPa gauge (3000 psig) in the presence of added hydrogen gas with the catalyst.

36. The process of Claim 35 wherein the zeolite is predominantly in the hydrogen form.

37. The process of Claim 35 wherein the catalyst further comprises at least one Group VIII metal.

38. The process of Claim 19 wherein the process is a process for isomerization dewaxing a raffinate comprising contacting said raffinate in the presence of added hydrogen under isomerization dewaxing conditions with the catalyst.

39. The process of Claim 38 wherein the zeolite is predominantly in the hydrogen form.

40. The process of Claim 38 wherein the catalyst further comprises at least one Group VIII metal.

41. The process of Claim 38 wherein the raffinate is bright stock.

42. The process of Claim 19 wherein the process is a process for increasing the octane of a hydrocarbon feedstock to produce a product having an increased aromatics content comprising contacting a hydrocarbonaceous feedstock which comprises normal and slightly branched hydrocarbons having a boiling range above about 40°C and less than about 200°C under aromatic conversion conditions with the catalyst.

43. The process of Claim 42 wherein the zeolite is substantially free of acid.

44. The process of Claim 42 wherein the zeolite contains a Group VIII metal component.

45. The process of Claim 19 wherein the process is a catalytic cracking process comprising contacting a hydrocarbon feedstock in a reaction zone under catalytic cracking conditions in the absence of added hydrogen with the catalyst.

46. The process of Claim 45 wherein the zeolite is predominantly in the hydrogen form.

47. The process of Claim 45 wherein the catalyst additionally comprises a large pore crystalline cracking component.

48. The process of Claim 19 wherein the process is an isomerization process for isomerizing C₄ to C₁ hydrocarbons, comprising contacting a feed having normal and slightly branched C₄ to C₇ hydrocarbons under isomerizing conditions with the catalyst.

49. The process of Claim 48 wherein the zeolite is predominantly in the hydrogen form.

50. The process of Claim 48 wherein the zeolite has been impregnated with at least one Group VIII metal.

51. The process of Claim 48 wherein the catalyst has been calcined in a steam/air mixture at an elevated temperature after impregnation of the Group VIII metal.

52. The process of Claim 50 wherein the Group VIII metal is platinum.

53. The process of Claim 19 wherein the process is a process for alkylating an aromatic hydrocarbon which comprises contacting under alkylation conditions at least a molar excess of an aromatic hydrocarbon with a C₂ to C₂₀ olefin under at least partial liquid phase conditions and in the presence of the catalyst.

54. The process of Claim 53 wherein the zeolite is predominantly in the hydrogen form.

55. The process of Claim 53 wherein the olefin is a C₂ to C₄ olefin.

56. The process of Claim 55 wherein the aromatic hydrocarbon and olefin are present in a molar ratio of about 4:1 to about 20:1, respectively.

57. The process of Claim 55 wherein the aromatic hydrocarbon is selected from the group consisting of benzene, toluene, ethylbenzene, xylene, naphthalene, dimethylnaphthalene, naphthalene derivatives or mixtures thereof.

58. The process of Claim 19 wherein the process is a process for transalkylating an aromatic hydrocarbon which comprises contacting under transalkylating conditions an aromatic hydrocarbon with a polyalkyl aromatic hydrocarbon under at least partial liquid phase conditions and in the presence of the catalyst.

59. The process of Claim 58 wherein the zeolite is predominantly in the hydrogen form.

60. The process of Claim 58 wherein, the aromatic hydrocarbon and the polyalkyl aromatic hydrocarbon are present in a molar ratio of from about 1:1 to about 25:1, respectively.

61. The process of Claim 58 wherein the aromatic hydrocarbon is selected from the group consisting of benzene, toluene, ethylbenzene, xylene, or mixtures thereof.

62. The process of Claim 58 wherein the polyalkyl aromatic hydrocarbon is a dialkylbenzene.

63. The process of Claim 19 wherein the process is a process to convert paraffins to aromatics which comprises contacting paraffins under conditions which cause paraffins to convert to aromatics with a catalyst comprising the zeolite and gallium, zinc, or a compound of gallium or zinc.

64. The process of Claim 19 wherein the process is a process for isomerizing olefins comprising contacting said olefin under conditions which cause isomerization of the olefin with the catalyst.

65. The process of Claim 19 wherein the process is a process for isomerizing an isomerization feed comprising an aromatic C₈ stream of xylene isomers or mixtures of xylene isomers and ethylbenzene, wherein a more nearly equilibrium ratio of ortho-, meta and para-xylenes is obtained, said process comprising contacting said feed under isomerization conditions with the catalyst.

66. The process of Claim 19 wherein the process is a process for oligomerizing olefins comprising contacting an olefin feed under oligomerization conditions with the catalyst.

67. A process for converting lower alcohols and other oxygenated hydrocarbons comprising contacting said lower alcohol or other oxygenated hydrocarbon under conditions to produce liquid products with a catalyst comprising the zeolite according to claim 1 or 2.

68. The process of Claim 19 wherein the process is a process for the production of higher molecular weight hydrocarbons from lower molecular weight hydrocarbons comprising the steps of:
(a) introducing into a reaction zone a lower molecular weight hydrocarbon-containing gas and contacting said gas in said zone under C₂+ hydrocarbon synthesis conditions with the catalyst and a metal or metal compound capable of converting the lower molecular weight hydrocarbon to a higher molecular weight hydrocarbon; and
(b) withdrawing from said reaction zone a higher molecular weight hydrocarbon-containing stream.

69. The process of Claim 68 wherein the metal or metal compound comprises a lanthanide or actinide metal or metal compound.

70. The process of Claim 68 wherein the lower molecular weight hydrocarbon is methane.

71. A process for the reduction of oxides of nitrogen contained in a gas stream in the presence of oxygen wherein said process comprises contacting the gas stream with the zeolite according to claim 1 or 2.

72. The process of Claim 71 wherein said zeolite contains a metal or metal ions capable of catalyzing the reduction of the oxides of nitrogen.

73. The process of Claims 72 wherein the metal is copper, or mixtures thereof.

74. The process of Claim 72 wherein the gas stream is the exhaust stream of an internal combustion engine.

## Patentansprüche

1. Zeolith, dessen Molverhältnis von einem Oxid eines ersten vierwertigen Elementes zu einem Oxid eines zweiten vierwertigen Element, das sich von dem ersten vierwertigen Element unterscheidet, eines dreiwertigen Elementes, fünfwertigen Elementes oder eines Gemischs davon, größer als etwa 20 ist und der nach der Kalzinierung die Röntgenbeugungslinien der nachstehenden Tabelle II aufweist:
**TABELLE II**
| kalzinierter SSZ-58 | | |
|---|---|---|
| 2 Theta (°)^{(a)} | d(Å/×10⁻¹⁰)m | relative Intensität |
| 7,1 | 12,4 | VS |
| 7,7 | 11,5 | M |
| 9,9 | 8,93 | M |
| 10,5 | 8,42 | M |
| 12,1 | 7,31 | W |
| 17,3 | 5,12 | W |
| 19,8 | 4,48 | M |
| 21,0 | 4,23 | S |
| 21,9 | 4,06 | M |
| 22,4 | 3,97 | S |
| | | |
|---|---|---|
| ^{(a)} ± 0,15 | | |

2. Zeolith nach Anspruch 1, dessen Molverhältnis von einem Oxid, ausgewählt aus der Gruppe Siliciumoxid, Germaniumoxid und Gemischen davon, zu einem Oxid, ausgewählt aus Aluminiumoxid, Galliumoxid, Eisenoxid, Boroxid, Titanoxid, Indiumoxid, Vanadiumoxid und Gemischen davon, größer als etwa 20 ist.

3. Zeolith nach Anspruch 2, wobei die Oxide Siliciumoxid und Aluminiumoxid umfassen.

4. Zeolith nach Anspruch 2, wobei die Oxide Siliciumoxid und Boroxid umfassen.

5. Zeolith nach Anspruch 1, welcher vorwiegend in der Wasserstoffform vorliegt.

6. Zeolith nach Anspruch 1, welcher im Wesentlichen keine Azidität aufweist.

7. Zeolith nach Anspruch 1 oder 2 mit einer Zusammensetzung, wie synthetisiert und im wasserfreien Zustand, ausgedrückt als Molverhältnisse wie folgt:
YO₂/W_{c}O_{d} >20
M_{2/n}/YO₂ 0,01 - 0,03
Q/YO₂ 0,02 - 0,05
wobei Y Silicium, Germanium oder ein Gemisch davon ist, W Aluminium, Gallium, Eisen, Bor, Titan, Indium, Vanadium oder Gemische davon ist, c gleich 1 oder 2 ist, d gleich 2 ist, wenn c gleich 1 ist oder d gleich 3 oder 5 ist, wenn c gleich 2 ist; M ein Alkalimetallkation, Erdalkalimetallkation oder Gemische davon ist; n die Wertigkeit von M angibt; und Q ein N-Butyl-N-cyclooctylpyrrolidinium-Kation oder N-Propylcyclooctylpyrrolidinium-Kation ist.

8. Zeolith nach Anspruch 7, wobei W Aluminium und Y Silicium ist.

9. Zeolith nach Anspruch 7, wobei W Bor und Y Silicium ist.

10. Zeolith nach Anspruch 7, wobei Q ein N-Butylcyclooctylpyrrolidinium-Kation ist.

11. Zeolith nach Anspruch 7, wobei Q ein N-Propylcyclooctylpyrrelidinium-Kation ist.

12. Verfahren zur Herstellung des Zeoliths nach Anspruch 1 oder 2, umfassend das Zusammenbringen von Quellen für diese Oxide und einem Matrizenmittel, welches ein N-Butyl-N-cyclooctylpyrrolidinium-Kation oder N-Propylcyclooctylpyrrolidinium-Kation umfasst, unter Kristallisationsbedingungen,

13. Verfahren nach Anspruch 12, wobei das erste vierwertige Element ausgewählt ist aus Silicium, Germanium und Kombinationen davon.

14. Verfahren nach Anspruch 12, wobei das zweite vierwertige, dreiwertige oder fünfwertige Element ausgewählt ist aus Aluminium, Gallium, Eisen, Bor, Titan, Indium, Vanadium und Kombinationen davon

15. Verfahren nach Anspruch 14, wobei das zweite vierwertige Element oder das dreiwertige Element ausgewählt ist aus Aluminium, Bor, Titan und Kombinationen davon.

16. Verfahren nach Anspruch 15, wobei das erste vierwertige Element Silicium ist.

17. Verfahren nach Anspruch 12, wobei das Matrizenmittel ein N-Butylcyclooctylpyrrolidinium-Kation ist.

18. Verfahren nach Anspruch 12, wobei das Matrizenmittel ein N-Propylcyclooctylpyrrolidinium-Kation ist.

19. Verfahren zum Umwandeln von Kohlenwasserstoffen, umfassend das Zusammenbringen einer Kohlenwasserstoff-Beschickung mit einem Katalysator, welcher den Zeolithen nach Anspruch 1 oder 2 umfasst, unter Kohlenwasserstoff-Umwandlungsbedingungen.

20. Verfahren nach Anspruch 19, wobei der Zeolith vorwiegend in der Wasserstoffform vorliegt.

21. Verfahren nach Anspruch 19, wobei der Zeolith im Wesentlichen keine Azidität aufweist.

22. Verfahren nach Anspruch 19, nämlich ein Hydrocrack-Verfahren, umfassend das Zusammenbringen des Katalysators mit einer Kohlenwasserstoff-Beschickung unter Hydrocrackbedingungen.

23. Verfahren nach Anspruch 22, wobei der Zeolith vorwiegend in der Wasserstoffform vorliegt.

24. Verfahren nach Anspruch 19, nämlich ein Entwachsungsverfahren, umfassend das Zusammenbringen des Katalysators mit einer Kohlenwasserstoff-Beschickung unter Entwachsungsbedingungen.

25. Verfahren nach Anspruch 24, wobei der Zeolith vorwiegend in der Wasserstoffform vorliegt.

26. Verfahren nach Anspruch 19, nämlich ein Verfahren zum Verbessern des Viskositätsindexes eines entwachsten Produktes wachsartiger Kohlenwasserstoff-Beschickungen, umfassend das Zusammenbringen des Katalysators mit einer wachsartigen Kohlenwasserstoff-Beschickung unter Isomerisierungs-Entwachsungsbedingungen.

27. Verfahren nach Anspruch 26, wobei der Zeolith vorwiegend in der Wasserstoffform vorliegt.

28. Verfahren nach Anspruch 19, nämlich ein Verfahren zur Herstellung eines C₂₀₊-Schmieröls aus einer C₂₀₊-Olefinbeschickung, umfassend das Isomerisieren der Olefinbeschickung über dem Katalysator unter Isomerisierungsbedingungen.

29. Verfahren nach Anspruch 28, wobei der Zeolith vorwiegend in der Wasserstoffform vorliegt.

30. Verfahren nach Anspruch 28, wobei der Katalysator zudem mindestens ein Gruppe-VIII-Metall umfasst.

31. Verfahren nach Anspruch 19, nämlich ein Verfahren zum katalytischen Entwachsen einer Kohlenwasserstofföl-Beschickung, die oberhalb von etwa 177°C (350°F) siedet und gerade und leicht verzweigte Kohlenwasserstoffketten enthält, umfassend das Zusammenbringen der Kohlenwasserstofföl-Beschickung mit dem Katalysator in Gegenwart von hinzugefügtem Wasserstoffgas bei einem Wasserstoffdruck von etwa 0,10 - 20,68 MPa (15 - 3000 psi) unter Entwachsungsbedingungen.

32. Verfahren nach Anspruch 31, wobei der Zeolith vorwiegend in der Wasserstoffform vorliegt.

33. Verfahren nach Anspruch 31, wobei der Katalysator zudem mindestens ein Gruppe-VIII-Metal umfasst.

34. Verfahren nach Anspruch 31, wobei der Katalysator einen schichtförmigen Katalysator umfasst mit einer ersten Schicht, welche den Zeolithen und mindestens ein Gruppe-VIII-Metall umfasst, und einer zweiten Schicht, welche einen Aluminosilicat-Zeolithen umfasst, der formselektiver als der Zeolith der ersten Schicht ist.

35. Verfahren nach Anspruch 19, nämlich ein Verfahren zur Herstellung eines Schmieröls, umfassend:
Hydrocracken einer Kohlenwasserstoff-Beschickung in einem Hydrocrack-Bereich, so dass ein Ausfluss erhalten wird, der ein Hydrocrack-ÖI enthält; und
katalytisches Entwachsen des Ausflusses, der das Hydrocrack-Öl enthält, bei einer Temperatur von mindestens etwa 204°C (400°F) und einem Druck von etwa 0,103 bis 20,7 MPa Druckanzeige (etwa 15 psig bis etwa 3000 psig) in Gegenwart von hinzu gefügtem Wasserstoffgas, mit dem Katalysator.

36. Verfahren nach Anspruch 35, wobei der Zeolith vorwiegend in der Wasserstoffform vorliegt.

37. Verfahren nach Anspruch 35, wobei der Katalysator zudem mindestens ein Gruppe-VIII-Metall umfasst.

38. Verfahren nach Anspruch 19, nämlich ein Verfahren zum Isomerisierungs-Entwachsen eines Raffinats, umfassend das Zusammenbringen des Raffinats mit dem Katalysator in Gegenwart von zugefügtem Wasserstoff unter Isomerisierungs-Entwachsungsbedingungen.

39. Verfahren nach Anspruch 38, wobei der Zeolith vorwiegend in der Wasserstoffform vorliegt.

40. Verfahren nach Anspruch 38, wobei der Katalysator zudem mindestens ein Gruppe-VIII-Metall umfasst.

41. Verfahren nach Anspruch 38, wobei das Raffinat Brightstock ist.

42. Verfahren nach Anspruch 19, nämlich ein Verfahren zum Erhöhen der Oktanzahl einer Kohlenwasserstoff-Beschickung, so dass ein Produkt mit einem größeren Aromatengehalt erzeugt wird, umfassend das Zusammenbringen einer Kohlenwasserstoff-Beschickung, welche normale und leicht verzweigte Kohlenwasserstoffe mit einem Siedebereich oberhalb von etwa 40°C und unterhalb von etwa 200°C umfasst, mit dem Katalysator unter Aromaten-Umwandlungsbedingungen.

43. Verfahren nach Anspruch 42, wobei der Zeolith im Wesentlichen säurefrei ist.

44. Verfahren nach Anspruch 42, wobei der Katalysator eine Gruppe-VIII-MetallKomponente umfasst.

45. Verfahren nach Anspruch 19, nämlich ein katalytisches Crack-Verfahren, umfassend das Zusammenbringen einer Kohlenwasserstoff-Beschickung mit dem Katalysator in einem Reaktionsbereich unter katalytischen Crackbedingungen in Abwesenheit von zugefügtem Wasserstoff.

46. Verfahren nach Anspruch 45, wobei der Zeolith vorwiegend in der Wasserstoffform vorliegt.

47. Verfahren nach Anspruch 45, wobei der Katalysator zudem eine großporige kristalline Crack-Komponente umfasst.

48. Verfahren nach Anspruch 19, nämlich ein lsomerisierungsverfahren zum Isomerisieren von C₄- bis C₇-Kohlenwasserstoffen, umfassend das Zusammenbringen einer Beschickung, die normale und leicht verzweigte C₄- bis C₇-Kohlenwasserstoffe enthält, mit dem Katalysator unter Isomerisierungsbedingungen.

49. Verfahren nach Anspruch 48, wobei der Zeolith vorwiegend in der Wasserstoffform vorliegt.

50. Verfahren nach Anspruch 48, wobei der Zeolith mit mindestens einem Gruppe-VIII-Metall diffusionsbeschichtet ist.

51. Verfahren nach Anspruch 48, wobei der Katalysator nach der Diffusionsbeschichtung mit dem Gruppe-VIII-Metall in einem Dampf-Luft-Gemisch bei erhöhter Temperatur kalziniert wird.

52. Verfahren nach Anspruch 50, wobei das Gruppe-VIII-Metall Platin ist.

53. Verfahren nach Anspruch 19, nämlich ein Verfahren zum Alkylieren eines aromatischen Kohlenwasserstoffes, umfassend das Zusammenbringen von mindestens einem molaren Überschuss eines aromatischen Kohlenwasserstoffes mit einem C₂- bis C₂₀-Olefin in Gegenwart des Katalysators unter Alkylierungsbedingungen sowie unter zumindest partiellen Flüssigphase-Bedingungen.

54. Verfahren nach Anspruch 53, wobei der Zeolith vorwiegend in der Wasserstoffform vorliegt.

55. Verfahren nach Anspruch 53, wobei das Olefin ein C₂- bis C₄-Olefin ist.

56. Verfahren nach Anspruch 55, wobei der aromatische Kohlenwasserstoff und das Olefin in einem Molverhältnis von etwa 4:1 bis etwa 20:1 vorliegen,

57. Verfahren nach Anspruch 55, wobei der aromatische Kohlenwasserstoff ausgewählt ist aus Benzol, Toluol, Ethylbenzol, Xylol, Naphthalin, Dimethylnaphthalin, Naphthalin-Derivaten oder Gemischen davon.

58. Verfahren nach Anspruch 19, nämlich ein Verfahren zum Transalkylieren eines aromatischen Kohlenwasserstoffes, umfassend das Zusammenbringen eines aromatischen Kohlenwasserstoffes mit einem polylalkylaromatischen Kohlenwasserstoff in Gegenwart des Katalysators unter Transalkylierungsbedingungen sowie unter zumindest partiellen Flüssigphase-Bedingungen.

59. Verfahren nach Anspruch 58, wobei der Zeolith vorwiegend in der Wasserstoffform vorliegt.

60. Verfahren nach Anspruch 58, wobei der aromatische Kohlenwasserstoff und der polyalkylaromatische Kohlenwasserstoff in einem Molverhältnis von etwa 1:1 bis zu etwa 25:1 vorliegen.

61. Verfahren nach Anspruch 58, wobei der aromatische Kohlenwasserstoff ausgewählt ist aus Benzol, Toluol, Ethylbenzol, Xylol oder Gemischen davon.

62. Verfahren nach Anspruch 58, wobei der polyalkylaromatische Kohlenwasserstoff ein Dialkylbenzol ist.

63. Verfahren nach Anspruch 19, nämlich ein Verfahren zur Umwandlung von Paraffinen in Aromaten, umfassend das Zusammenbringen von Paraffinen mit einem Katalysator, welcher den Zeolithen und Gallium, Zink oder eine Gallium-oder Zinkverbindung umfasst, unter Bedingungen, bei denen Paraffine in Aromaten umgewandelt werden.

64. Verfahren nach Anspruch 19, nämlich ein Verfahren zum Isomerisieren von Olefinen, umfassend das Zusammenbringen des Olefins mit dem Katalysator unter Bedingungen, die die Isomerisierung des Olefins bewirken.

65. Verfahren nach Anspruch 19, nämlich ein Verfahren zum Isomerisieren einer Isomerisierungsbeschickung, welche einen aromatischen C₈-Strom von Xylolisomeren oder Gemische von Xylolisomeren und Ethylbenzol umfasst, wobei ein Verhältnis von ortho-, meta- und para-Xylolen erhalten wird, das noch näher am Gleichgewicht ist, umfassend das Zusammenbringen der Beschickung mit dem Katalysator unter Isomerisierungsbedingungen.

66. Verfahren nach Anspruch 19, nämlich ein Verfahren zum Oligomerisieren von Olefinen, umfassend das Zusammenbringen einer Olefinbeschickung mit dem Katalysator unter Oligomerisierungsbedingungen.

67. Verfahren zum Umwandeln von Niederalkoholen und anderen oxygenierten Kohlenwasserstoffen, umfassend das Zusammenbringen des Niederalkohols oder anderen oxygenierten Kohlenwasserstoffs mit einem Katalysator, umfassend den Zeolithen nach Anspruch 1 oder 2, unter Bedingungen, dass flüssige Produkte erhalten werden

68. Verfahren nach Anspruch 19, nämlich ein Verfahren zur Produktion von höhermolekularen Kohlenwasserstoffen aus niedermolekularen Kohlenwasserstoffen, umfassend die Schritte:
(a) Einführen eines niedermolekularen kohlenwasserstoffhaltigen Gases in einen Reaktionsbereich, und Zusammenbringen des Gases in dem Bereich unter C₂₊-Kohlenwasserstoff-Synthesebedingungen mit dem Katalysator und einem Metall oder einer Metallverbindung, die den niedermolekularen Kohlenwasserstoff in einen höhermolekularen Kohlenwasserstoff umwandeln kann; und
(b) Entnehmen eines höhermolekularen Kohlenwasserstoffstroms aus dem Reaktionsbereich.

69. Verfahren nach Anspruch 68, wobei das Metall oder die Metallverbindung ein Lanthaniden- oder Aktinidenmetall oder eine -Metallverbindung umfasst.

70. Verfahren nach Anspruch 68, wobei der niedermolekulare Kohlenwasserstoff Methan ist.

71. Verfahren zum Reduzieren der in einem Gasstrom enthaltenen Stickoxide in Gegenwart von Sauerstoff, umfassend das Zusammenbringen des Gasstroms mit einem Zeolithen nach Anspruch 1 oder 2.

72. Verfahren nach Anspruch 71, wobei der Zeolith ein Metall oder Metallionen enthält, welche die Reduktion der Stickoxide katalysieren können.

73. Verfahren nach Anspruch 72, wobei das Metall Kupfer, Kobalt oder Gemische davon ist.

74. Verfahren nach Anspruch 72, wobei der Gasstrom der Abgasstrom eines Verbrennungsmotors ist.

## Revendications

1. Zéolite ayant un rapport molaire supérieur à environ 20 d'un oxyde d'un premier élément tétravalent à un oxyde d'un second élément tétravalent qui est différent dudit premier élément tétravalent, élément trivalent, élément pentavalent ou mélange d'entre eux et ayant, après calcination, les raies de diffraction des rayons X du Tableau II ci-dessous :
**TABLEAU II**
| SSZ-58 calcinée | | |
|---|---|---|
| 2 thêta (deg.) ^{(a)} | d (Å/×10⁻¹⁰)m | Intensité relative |
| 7,1 | 12,4 | Très intense |
| 7,7 | 11,5 | Moyenne |
| 9,9 | 8,93 | Moyenne |
| 10,5 | 8,42 | Moyenne |
| 12,1 | 7,31 | Faible |
| 17,3 | 5,12 | Faible |
| 19,8 | 4,48 | Moyenne |
| 21,0 | 4,23 | Intense |
| 21,9 | 4,06 | Moyenne |
| 22,4 | 3,97 | Intense |
| | | |
|---|---|---|
| ^{(a)}±0,15 | | |

2. Zéolite selon la revendication 1, ayant un rapport molaire supérieur à environ 20 d'un oxyde choisi dans le groupe formé par l'oxyde de silicium, l'oxyde de germanium et leurs mélanges à un oxyde choisi dans le groupe formé par l'oxyde d'aluminium, l'oxyde de gallium, l'oxyde de fer, l'oxyde de bore, l'oxyde de titane, l'oxyde d'indium, l'oxyde de vanadium et leurs mélanges.

3. Zéolite selon la revendication 2, dans laquelle les oxydes comprennent l'oxyde de silicium et l'oxyde d'aluminium.

4. Zéolite selon la revendication 2, dans laquelle les oxydes comprennent l'oxyde de silicium et l'oxyde de bore.

5. Zéolite selon la revendication 1, dans laquelle ladite zéolite est principalement sous la forme hydrogène.

6. Zéolite selon la revendication 1, dans laquelle ladite zéolite est sensiblement dépourvue d'acidité.

7. zéolite selon la revendication 1 ou 2, ayant une composition, telle que synthétisée et à l'état anhydre, exprimée en rapports molaires, comme suit :
YO₂/W_{c}O_{d} >20
M_{2/n}/YO₂ 0,01 - 0,03
Q/YO₂ 0,02 - 0,05
où Y est le silicium, le germanium, ou un mélange d'entre eux ; W est l'aluminium, le gallium, le fer, le bore, le titane, l'indium, le vanadium ou un mélange d'entre eux ; c est 1 ou 2 ; d est 2 lorsque c est 1 ou bien d est 3 ou 5 lorsque c est 2 ; M est un cation de métal alcalin, un cation de métal alcalino-terreux ou un mélange d'entre eux ; n est la valence de M ; et Q est un cation N-butyl-N-cyclooctylpyrrolidinium ou un cation N-propyl-cyclooctylpyrrolidinium.

8. Zéolite selon la revendication 7, dans laquelle W est l'aluminium et Y est le silicium.

9. Zéolite selon la revendication 7, dans laquelle W est le bore et Y est le silicium.

10. Zéolite selon la revendication 7, dans laquelle Q est un cation N-butyl-cyclooctylpyrrolidinium.

11. Zéolite selon la revendication 7, dans laquelle est un cation N-propyl-cyclooctylpyrrolidinium.

12. Procédé de préparation de la zéolite de la revendication 1 ou 2, comprenant la mise en contact, dans des conditions de cristallisation, de sources desdits oxydes et d'un agent structurant comprenant un cation N-butyl-N-cyclooctylpyrrolidinium ou un cation N-propyl-cyclooctylpyrrolidinium.

13. Procédé selon la revendication 12, dans lequel le premier élément tétravalent est choisi dans le groupe formé par le silicium, le germanium et leurs associations.

14. Procédé selon la revendication 12, dans lequel le second élément tétravalent, élément trivalent ou élément pentavalent est choisi dans le groupe formé par l'aluminium, le gallium, le fer, le bore, le titane, l'indium, le vanadium et leurs associations.

15. Procédé selon la revendication 14, dans lequel le second élément tétravalent ou élément trivalent est choisi dans le groupe formé par l'aluminium, le bore, le titane et leurs associations.

16. Procédé selon la revendication 15, dans lequel le premier élément tétravalent est le silicium.

17. Procédé selon la revendication 12, dans lequel l'agent structurant est un cation N-butyl-cyclooctylpyrrolidinium.

18. Procédé selon la revendication 12, dans lequel l'agent structurant est un cation N-propyl-cyclooctylpyrrolidinium.

19. Procédé de conversion d'hydrocarbures, comprenant la mise en contact d'une charge d'alimentation d'hydrocarbures dans des conditions de conversion d'hydrocarbures avec un catalyseur comprenant la zéolite selon la revendication 1 ou 2.

20. Procédé selon la revendication 19, dans lequel la zéolite est principalement sous la forme hydrogène.

21. Procédé selon la revendication 19, dans lequel la zéolite est sensiblement dépourvue d'acidité.

22. Procédé selon la revendication 19, dans lequel le procédé est un procédé d'hydrocraquage comprenant la mise en contact du catalyseur avec une charge d'hydrocarbures dans des conditions d'hydrocraquage.

23. Procédé selon la revendication 22, dans lequel la zéolite est principalement sous la forme hydrogène.

24. Procédé selon la revendication 19, dans lequel le procédé est un procédé de déparaffinage comprenant la mise en contact du catalyseur avec une charge d'hydrocarbures dans des conditions de déparaffinage.

25. Procédé selon la revendication 24, dans lequel la zéolite est principalement sous la forme hydrogène.

26. Procédé selon la revendication 19, dans lequel le procédé est un procédé pour améliorer l'indice de viscosité d'un produit déparaffiné de charges d'hydrocarbures paraffiniques, comprenant la mise en contact du catalyseur avec une charge d'hydrocarbures paraffinique dans des conditions de déparaffinage par isomérisation.

27. Procédé selon la revendication 26, dans lequel la zéolite est principalement sous la forme hydrogène.

28. Procédé selon la revendication 19, dans lequel le procédé est un procédé pour la production d'une huile lubrifiante en C₂₀₊ à partir d'une charge d'oléfines en C₂₀₊, comprenant l'isomérisation de ladite charge d'oléfines dans des conditions d'isomérisation sur le catalyseur.

29. Procédé selon la revendication 28, dans lequel la zéolite est principalement sous la forme hydrogène.

30. Procédé selon la revendication 28, dans lequel le catalyseur comprend de plus au moins un métal du Groupe VIII.

31. Procédé selon la revendication 19, dans lequel le procédé est un procédé pour le déparaffinage catalytique d'une charge d'huile d'hydrocarbures bouillant au-dessus d'environ 177°C (350°F) et contenant des hydrocarbures à chaîne droite et à chaîne légèrement ramifiée, comprenant la mise en contact avec le catalyseur de ladite charge d'huile d'hydrocarbures en présence d'hydrogène gazeux ajouté à une pression d'hydrogène d'environ 15 à 3000 psi (0,10 à 20,68 MPa), dans des conditions de déparaffinage.

32. Procédé selon la revendication 31, dans lequel la zéolite est principalement sous la forme hydrogène.

33. Procédé selon la revendication 31, dans lequel le catalyseur comprend de plus au moins un métal du Groupe VIII.

34. Procédé selon la revendication 31, dans lequel ledit catalyseur comprend un catalyseur stratifié comprenant une première couche comprenant la zéolite et au moins un métal du Groupe VIII, et une seconde couche comprenant une zéolite d'aluminosilicate qui est plus sélective envers la forme que la zéolite de ladite première couche.

35. Procédé selon la revendication 19, dans lequel le procédé est un procédé pour la préparation d'une huile lubrifiante, qui comprend :
l'hydrocraquage, dans une zone d'hydrocraquage, d'une charge d'alimentation d'hydrocarbures pour produire un effluent comprenant une huile hydrocraquée ; et
le déparaffinage catalytique avec le catalyseur dudit effluent comprenant une huile hydrocraquée, à une température d'au moins environ 204°C (400°F) et à une pression d'environ 0,103 MPa au manomètre (15 psig) à environ 20,7 MPa au manomètre (3000 psig) en présence d'hydrogène gazeux ajouté.

36. Procédé selon la revendication 35, dans lequel la zéolite est principalement sous la forme hydrogène.

37. Procédé selon la revendication 35, dans lequel le catalyseur comprend de plus au moins un métal du Groupe VIII.

38. Procédé selon la revendication 19, dans lequel le procédé est un procédé de déparaffinage par isomérisation d'un raffinat, comprenant la mise en contact dudit raffinat avec le catalyseur, dans des conditions de déparaffinage par isomérisation, en présence d'hydrogène ajouté.

39. Procédé selon la revendication 38, dans lequel la zéolite est principalement sous la forme hydrogène.

40. Procédé selon la revendication 38, dans lequel le catalyseur comprend de plus au moins un métal du Groupe VIII.

41. Procédé selon la revendication 38, dans lequel le raffinat est une base lubrifiante de haute viscosité.

42. Procédé selon la revendication 19, dans lequel le procédé est un procédé pour augmenter l'indice d' octane d'une charge d'hydrocarbures pour produire un produit ayant une teneur accrue en composés aromatiques, comprenant la mise en contact avec le catalyseur d'une charge d'alimentation d'hydrocarbures comprenant des hydrocarbures normaux et légèrement ramifiés ayant un intervalle d'ébullition supérieur à environ 40°C et inférieur à environ 200°C, dans des conditions de conversion en composés aromatiques.

43. Procédé selon la revendication 42, dans lequel la zéolite est sensiblement dépourvue d'acide.

44. Procédé selon la revendication 42, dans lequel la zéolite contient un composant métallique du Groupe VIII.

45. Procédé selon la revendication 19, dans lequel le procédé est un procédé de craquage catalytique comprenant la mise en contact d'une charge d'alimentation d'hydrocarbures avec le catalyseur dans une zone de réaction dans des conditions de craquage catalytique en l'absence d'hydrogène ajouté.

46. Procédé selon la revendication 45, dans lequel la zéolite est principalement sous la forme hydrogène.

47. Procédé selon la revendication 45, dans lequel le catalyseur comprend de plus un composant de craquage cristallin à grands pores.

48. Procédé selon la revendication 19, dans lequel le procédé est un procédé d'isomérisation pour isomériser des hydrocarbures en C₄ à C₇, comprenant la mise en contact avec le catalyseur d'une charge contenant des hydrocarbures en C₄ à C₇ normaux et légèrement ramifiés, dans des conditions d'isomérisation.

49. Procédé selon la revendication 48, dans lequel la zéolite est principalement sous la forme hydrogène.

50. Procédé selon la revendication 48, dans lequel la zéolite a été imprégnée avec au moins un métal du Groupe VIII.

51. Procédé selon la revendication 48, dans lequel le catalyseur a été calciné dans un mélange de vapeur d'eau/air à une température élevée après l'imprégnation avec le métal du Groupe VIII.

52. Procédé selon la revendication 50, dans lequel le métal du Groupe VIII est le platine.

53. Procédé selon la revendication 19, dans lequel le procédé est un procédé pour alkyler un hydrocarbure aromatique, qui comprend la mise en contact, dans des conditions d'alkylation, d'au moins un excès molaire d'un hydrocarbure aromatique avec une oléfine en C₂ à C₂₀ dans des conditions de phase liquide au moins partielle et en présence du catalyseur.

54. Procédé selon la revendication 53, dans lequel la zéolite est principalement sous la forme hydrogène.

55. Procédé selon la revendication 53, dans lequel l'oléfine est une oléfine en C₂ à C₄.

56. Procédé selon la revendication 55, dans lequel l'hydrocarbure aromatique et l'oléfine sont présents en un rapport molaire d'environ 4:1 à environ 20:1, respectivement.

57. Procédé selon la revendication 55, dans lequel l'hydrocarbure aromatique est choisi dans le groupe formé par le benzène, le toluène, l'éthylbenzène, le xylène, le naphtalène, le diméthylnaphtalène, les dérivés du naphtalène ou leurs mélanges.

58. Procédé selon la revendication 19, dans lequel le procédé est un procédé de transalkylation d'un hydrocarbure aromatique, qui comprend la mise en contact, dans des conditions de transalkylation, d'un hydrocarbure aromatique avec un hydrocarbure aromatique polyalkylé dans des conditions de phase liquide au moins partielle et en présence du catalyseur.

59. Procédé selon la revendication 58, dans lequel la zéolite est principalement sous la forme hydrogène.

60. Procédé selon la revendication 58, dans lequel l'hydrocarbure aromatique et l'hydrocarbure aromatique polyalkylé sont présents en un rapport molaire d'environ 1:1 à environ 25:1, respectivement.

61. Procédé selon la revendication 58, dans lequel l'hydrocarbure aromatique est choisi dans le groupe formé par le benzène, le toluène, l'éthylbenzène, le xylène ou leurs mélanges.

62. Procédé selon la revendication 58, dans lequel l'hydrocarbure aromatique polyalkylé est un dialkylbenzène.

63. Procédé selon la revendication 19, dans lequel le procédé est un procédé pour convertir des paraffines en composés aromatiques, qui comprend la mise en contact de paraffines, dans des conditions provoquant la conversion des paraffines en composés aromatiques, avec un catalyseur comprenant la zéolite et du gallium, du zinc, ou un composé de gallium ou de zinc.

64. Procédé selon la revendication 19, dans lequel le procédé est un procédé pour isomériser des oléfines, comprenant la mise en contact de ladite oléfine avec le catalyseur dans des conditions provoquant l'isomérisation de l'oléfine.

65. Procédé selon la revendication 19, dans lequel le procédé est un procédé pour isomériser une charge d'isomérisation comprenant un courant aromatique en C₈ d'isomères de xylène ou de mélanges d'isomères de xylène et d'éthylbenzène, dans lequel on obtient un rapport plus proche de l'équilibre des *ortho-, méta-* et *para*-xylènes, ledit procédé comprenant la mise en contact de ladite charge avec le catalyseur dans des conditions d'isomérisation.

66. Procédé selon la revendication 19, dans lequel le procédé est un procédé d'oligomérisation d'oléfines, comprenant la mise en contact d'une charge d'oléfines avec le catalyseur dans des conditions d'oligomérisation.

67. Procédé de conversion d'alcools inférieurs et d'autres hydrocarbures oxygénés, comprenant la mise en contact dudit alcool inférieur ou autre hydrocarbure oxygéné, dans des conditions de production de produits liquides, avec un catalyseur comprenant la zéolite selon la revendication 1 ou 2.

68. Procédé selon la revendication 19, dans lequel le procédé est un procédé pour la production d'hydrocarbures de poids moléculaire supérieur à partir d'hydrocarbures de poids moléculaire inférieur, comprenant les étapes consistant à :
(a) introduire, dans une zone de réaction, un gaz contenant un hydrocarbure de poids moléculaire inférieur et mettre en contact ledit gaz dans ladite zone dans des conditions de synthèse d'hydrocarbures en C₂₊ avec le catalyseur et un métal ou composé de métal capable de convertir l'hydrocarbure de poids moléculaire inférieur en un hydrocarbure de poids moléculaire supérieur ; et
(b) retirer de ladite zone de réaction un courant contenant un hydrocarbure de poids moléculaire supérieur.

69. Procédé selon la revendication 68, dans lequel le métal ou composé de métal comprend un métal ou composé de métal de la série des lanthanides ou des actinides.

70. Procédé selon la revendication 68, dans lequel l'hydrocarbure de poids moléculaire inférieur est le méthane.

71. Procédé pour la réduction d'oxydes d'azote contenus dans un courant gazeux en présence d'oxygène, dans lequel ledit procédé comprend la mise en contact du courant gazeux avec la zéolite selon la revendication 1 ou 2.

72. Procédé selon la revendication 71, dans lequel ladite zéolite contient un métal ou des ions métalliques capables de catalyser la réduction des oxydes d'azote.

73. Procédé selon la revendication 72, dans lequel le métal est le cuivre, le cobalt ou un mélange d'entre eux.

74. Procédé selon la revendication 72, dans lequel le courant gazeux est le courant d'échappement d'un moteur à combustion interne.
